Europäisches Patentamt

European Patent Office

Office européen des brevets

(19)

(11) **EP 1 698 358 A1**

(12) **EUROPEAN PATENT APPLICATION**
published in accordance with Art. 158(3) EPC

(43) Date of publication:
06.09.2006 Bulletin 2006/36

(51) Int Cl.:
*A61L 27/40* (2006.01)  *A61L 27/38* (2006.01)
*A61F 2/06* (2006.01)  *A61F 2/24* (2006.01)
*A61F 2/28* (2006.01)  *C12N 5/06* (2006.01)
*C12N 5/08* (2006.01)

(21) Application number: 04807796.0

(22) Date of filing: 24.12.2004

(86) International application number:
PCT/JP2004/019440

(87) International publication number:
WO 2005/063316 (14.07.2005 Gazette 2005/28)

(84) Designated Contracting States:
AT BE BG CH CY CZ DE DK EE ES FI FR GB GR
HU IE IS IT LI LT LU MC NL PL PT RO SE SI SK TR

(30) Priority: 26.12.2003 JP 2003435945

(71) Applicants:
• Cardio Incorporated
  Osaka-shi, Osaka 530-0043 (JP)
• Osaka Prefecture University Public Corporation
  Osaka 599-8531 (JP)

(72) Inventors:
• MATSUDA, Hikaru,
  c/o E1 Osaka University Graduate
  Suita-shi,
  Osaka 565-0871 (JP)
• SAWA, Yoshiki,
  c/o E1 Osaka University Graduate
  Suita-shi,
  Osaka 565-0871 (JP)

• TAKETANI, Satoshi,
  E1 Osaka University Graduate
  Suita-shi, Osaka 565-0871 (JP)
• MIYAGAWA, Shigeru,
  E1 Osaka University Graduate
  Suita-shi, Osaka 5650871 (JP)
• IWAI, Shigemitsu,
  c/o E1 Osaka University
  Suita-shi, Osaka 565-0871 (JP)
• OTA, Takeyoshi,
  c/o E1 Osaka University
  Suita-shi, Osaka 565-0871 (JP)
• HARA, Masayuki
  Sakai-shi, Osaka 599-8570 (JP)
• FURUTA, Masakazu
  Sakai-shi, Osaka 599-8570 (JP)

(74) Representative: Khoo, Chong-Yee
  D Young & Co
  120 Holborn
  London EC1N 2DY (GB)

(54) **TRANSPLANTABLE BIOMATERIAL AND METHOD OF PREPARING THE SAME**

(57) Decellularized tissues prepared by conventional technology are occasionally to be improved with respect to the strength, etc. thereof according to situations. The reason is that cells are removed from tissues by the decellularization, occasionally resulting in poor strength of the tissues. It is intended to provide a method of increasing the tissue strength for the decellularized tissues. There is provided a method comprising treating the decellularized tissues with a biocompatible polymer. Unexpectedly strong reinforcing effect for the decellularized tissues by a biocompatible polymer, preferably one polymerized through radical reaction using gamma rays, etc. has been found. Decellularization can be performed by the use of arbitrary technique, for example, using a surfactant such as SDS, or using an amphiphilic molecule such as PEG.

EP 1 698 358 A1

**Description**

TECHNICAL FIELD

The present invention relates to a method and system for decellularizing tissue, tissue prepared by the decellularization method, and a medicament and therapeutic method utilizing a tissue graft or the like.

BACKGROUND ART

Implantation of organs (e.g., heart, blood vessel, etc.) derived from exogenous tissue is mainly hindered by immunological rejections. Changes occurring in allografts and xenografts were first described 90 years or more ago (Carrel A. , 1907, J. Exp. Med. 9:226-8; Carrel A., 1912., J. Exp. Med. 9:389-92; Guthrie C.C., 1908, J. Am. Med. Assoc; Calne R. Y., 1970, Transplant Proc. 2:550; and Auchincloss 1988, Transplantation 46:1). Rejections to artery grafts pathologically leads either to enlargement (up to rupture) or obstruction of the grafts. The former is caused by decomposition of the extracellular matrices, while the latter is caused by proliferation of cells within a blood vessel (Uretsky B. F., Mulari S., Reddy S. , et al. , 1987, Circulation 76:827-34).

Conventionally, two strategies have been used to alleviate rejection of these substances. One of the two strategies is to reduce the hosts immune reaction (Schmitz-Rixen T., Megerman J., Colvin R. B., Williams A. M., Abbot W., 1988, J. Vasc. Surg. 7:82-92; and Plissonnier D. , et al., 1993, Arteriosclerosis Thromb, 13:112-9) . The other strategy is to reduce the antigenicity of allografts or xenografts, mainly by cross-linking (Rosenberg N., et al., 1956, Surg. Forum 6:242-6; and Dumont C., Pissonnier D., Michel J. B., 1993, J. Surg. Res. 54:61-69). The cross-linking of extracellular matrices reduces the antigenicity of grafts, but changes bioengineering functions (Cosgrove D. M., Lytle B. W. , Golding C. C., et al., 1983, J. Thorac. Cardiovasc. Surgery 64:172-176; and Broom N., Christie G. W. , 1982, In: Cohn L. H., Gallucci V., editors. Cardiac bioprostheses: Proceedings of the Second International Symposium. New York: York Medical Books Pages 476-491), so that the grafts become susceptible to mineralization (Schoen F. J., Levy R. J., Piehler H. R., 1992, Cardiovasc. Pathology 1992; 1:29-52).

Cells in the extracellular matrices have Class I and II histocompatibility antigens capable of eliciting rejection reactions. Also, the cells have glycoprotelns recognized by the immune system of hosts, which elicit rejection reactions. Therefore, if these substances are eliminated from extracellular matrices, rejection reactions can be prevented. However, complete elimination of all antigens is considerably difficult to perform and verify. Malone et al. (Malone J. M., Brendel K., Duhamel R. C., Reinert R. L. , 1984, J. Vasc. Surg. 1:181-91) and Lalka et al. (Lalka S. G., Oelker L. M., and Malone J. M., et al., 1989, Ann Vasc. Surg., 3:108-17) reported that although immune reactions were stimulated in matrices to which "cell-free" artery allografts (graft to the same species animal) were implanted, proliferation in blood vessels and acceptance of endothelial cells were also observed. Most recently, O'Brian et al. reported that decellularized porcine tissue can be applied to implantation of cardiac blood vessels and that no hyperacute rejection was elicited when implanted into sheep (O' Brien M. F., et al., 1999 (October), Seminars in Thorac. and Cardiovasc. Surg.; 111(4), Suppl 1:194-200).

Cardiovascular disease, including coronary artery and peripheral vascular disease, is primarily treated by surgical replacement. The number of cardiovascular disease cases is presently increasing all over the world. In the case of small-diameter blood vessels, it is difficult to apply replacement therapy thereto. In the case of small diameter blood vessels, bypass operations are applied, where an autologous venous or arterial graft is used (Canver C. C., 1995, Chest 1995:108 1150-1155; Barner H. B. , 1998, Ann. Thorac. Surg., 66 (Suppl 5) S2-5, discussion S25-28; and Bhan A., Gupta V., Choudhary S. K., et al., 1999, Ann Thorac. Surg., 1999:67 1631-1636). Although venous and arterial grafts currently yield the best results, disadvantages include the need for complicated operations and no suitable blood vessels available in patients with certain diseases. As a result, there is a demand for a vascular prosthesis, which is suited to the small-diameter blood vessels. In order to reduce the use of auto- or allo-grafts, efforts have been made to develop artificial materials. However, no artificial material is suitable for construction of small-diameter arteries (<6 mm) required for extremity and coronary artery bypass grafting operations. On the other hand, in the case of cardiac blood vessels, the feasibility of native tissue grafts as biomaterials in clinical applications have been investigated. The use of xenograft and allograft tissues typically requires chemical or physical treatment (e.g., glutaraldehyde fixation). Cross-linking techniques have been investigated and found to be an ideal procedure for stabilizing the collagen-based structure of tissue (Hilbert S. L., Ferrans V. J., Jone M., 1988, Med Prog. Technol. 89:14, 115-163).

However, implantation have problems associated with calcification when viewed in the long term. This detrimental side effect of calcification associated in glutaraldehyde treatment is the main cause of failure of bio-prosthetic heart valves (Rao K.P., Shanthi C., 1999, Biomaterials Appl., 13:238-268; and Grabenwoger M., Sider J. , Fitzal F., et al. , 1996, Ann. Thorac. Surg., 62:772-777). As an alternative approach to native tissue grafts, an attempt has been made to produce an acellular tissue matrix by specifically removing cellular components which were believed to promote calcification and elicit an immune response. The decellularization technique includes chemical, enzymatic, and mechanical means for removing cellular components. This treatment leaves a material composed essentially of extracellular matrix components. The resultant decellularized tissue retains native mechanical properties and promotes regeneration. Regeneration is caused by neovasoularization and recellularization by the host. Surfactant treatment, which is a typical cell extraction method, has been carried out as a means for creating completely decellularized tissues for use as a biomaterial graft. This is because cellular components, lipids and residual surfactant remaining within treated tissue may promote undesired effects, such as calcification (Valente M. , Bortolotti U. , Thiene G. , 1985, Am. J. Pathol. 119, 12-21; Maranto A. R., Shoen F. J. , 1988, ASAIO Trans. 34, 827-830; Courtman D. W., Pereira C. A., Kashef V., McComb D., Lee J. M., Wilson G. L., 1994, J. Biomed. Mater. Res. 28:655-666; andLevyR. J., SchoenF. J., Anderson H. C., et al., 1991, Biomaterials 12:707-714). Removal of lipids from bovine pericardium treated by either chloroform/ methanol or sodium dodecyl sulfate (SDS) has been found to decrease calcification of tissues in a rat model (Jorge-Herrero E., Fernandez P., Gutierrez M. P., Castillo-Olivares J. L., 1991, Biomaterials 12:683-689). Recently, Sunjay et al. demonstrated that decellularized blood vessel grafts were coated with endothelial progenitor cells (EPCs). This is because these grafts have well preserved extracellular matrices and mechanical properties similar to those of native blood vessels, including arteries (Sunjay Kaushal, Gilad E. Amiel, Kristine J., Guleserian, et al., 2001, Nature Medicine Vol. 9, 1035-1040). Sunjay et al. isolated endothelial progenitor cells (EPCs) from peripheral blood and disseminated EPCs in decellularized bovine iliac blood vessels. The EPC-disseminated decellularized graft developed new blood vessels *in vivo* by day 130. The new blood vessels underwent NO-mediated vascular relaxation.

The present inventors have found that the use of an amphipathic solution unexpectedly improves decellularization efficiency, and in addition, have found that it has good biocompatibility and biological fixation properties. However , there are some occasions where tissues must be reinforced, and thus there is still a demand in the art for improvement in decellularization tissue.

[Patent Literature 1]
Japanese Laid-Open Publication No. 2002-543950
[Patent Literature 2]

Japanese Laid-Open Publication No. 2001-78750 号
[Patent Literature 3]
WO89/05371
[Non-Patent Literature 1]
Carrel A.,1907,J Exp Med 9:226-8
[Non-Patent Literature 2]
Carrel A.,1912.,J Exp Med 9:389-92
[Non-Patent Literature 3]
Toshiharu SHIN'OKA, Yasuharu IMAI, Kazuhiro SEO et al., "Tisshu enjiniaring ni yoru shin kekkan zairyo no kaihatsu, oyo. [Development and application of cardiovascular material by means of tissue engineering], Nichishinzoukekkangaikaishi. [Journal of Japanese Society for Cadiovascular Surgery] 2000;29:38.
[non-patent literature 4]
Calne RY.,1970,Transplant Proc 2:550
[non-patent literature 5]
Auchincloss 1988,Transplantation 46:1
[Non-Patent Literature 6]
Uretsky BF,Mulari S,Reddy S,et al., 1987, Circulation 76:827-34.
[Non-Patent Literature 7]
Schmitz-Rixen T,Megerman J,Colvin RB,Williams AM,Abbot W., 1988, J Vasc Surg 7:82-92.
[Non-Patent Literature 8]
Plissonnier D, et al., 1993, Arteriosclerosis Thromb 13:112-9
[Non-Patent Literature 9]
Rosenbexg N,et al.,1956,Surg Forum 6:242-6.
[Non-Patent Literature 10]
Dumont C,Pissonnier D,Michel JB.,1993,J Surg Res 54:61-69.
[Non-Patent Literature 11]
Cosgrovo DM,Lytle BW,Golding CC,et al.,1983,J Thorac Cardiovasc Surgery 64:172-1'76.
[Non-Patent Literature 12]
Broom N, Christie GW.,1982,In:Cohn LH,Gallucci V,editors.Cardiac bioprostheses:Proceedings of the Second International Symposium.New York: York Medical Books Pages 4'76-491.
[Non-Patent Literature 13]
Schoen FJ,Levy RJ,Piehler HR.,Cardiovasc Pathology 1992;1:29-52.

[Non-Patent Literature 14]

J Thorac Cardiovasc Surg 1998;115;536-46.

[Non-Patent Literature 15]

Malone JM,Brendel K,Duhamel RC,Reinert RL. ,1984, J Vasc Surg 1:181-91.

[Non-Patent Literature 16]

Lalka SG, Oelker LM, and Malone JM, et al., 1989, Ann Vasc Surg 3:108-17.

[Non-Patent Literature 17]

O'Brien MF,et al., 1999 (October), Seminars in Thorac and Cardiovasc Surg;111(4),Suppl 1:194-200.

[Non-Patent Literature 18]

Canver CC.,1995,Chest 1995;108 1150-1155.

[Non-Patent Literature 19]

Barner HB.,1998,Ann Thorac Surg 66(Suppl 5)S2-5;discussion S25-28.

[Non-Patent Literature 20]

Bhan A,Gupta V,Choudhary SK,etal.,1999,Ann Thorac Surg 1999;67 1631-1636).

[Non-Patent Literature 21]

Hilbert SL,Ferrans VJ,Jone M.,1988,Med Prog Technol 89;14,115-163.

[Non-Patent Literature 22]

Rao KP,Shanthi C.,1999,Biomatrials Appl 13:238-268

[Non-Patent Literature 23]

Grabenwoger M, Sider J, Fitzal F, et al., 1996, Ann Thorac Surg 62:772-777.

[Non-Patent Literature 24]

Valente M, Bortolotti U, Thiene G,, 1985, Am J Pathol 119, 12-21.

[Non-Patent Literature 25]

Maranto AR,Shoen FJ,1988,ASAIO Trans 34,827-830.

[Non-Patent Literature 26]

Courtman DW,Pereira CA,Kashef V,McComb D,Lee JM,Wilson GL,1994,J Biomed Mater Res 28:655-666.

[Non-Patent Literature 27]

Levy RJ,Schoen FJ,Anderson HC,et al.,1991,Biomaterials 12:'70'7-714.

[Non-Patent Literature 28]

Jorge-Herrero E, Fernandez P, Gutierrez MP, Castillo-Olivares JL,1991,Biomaterials 12:683-689.

[Non-Patent Literature 29]

Sunjay Kaushal,Gilad E.Amiel,Kristine J.Guleserian,et al.2001. Nature Medicine Vol.9,1035-1040.

DISCLOSURE OF THE INVENTION

PROBLEMS TO BE SOLVED BY THE INVENTION

There are some occasions where decellularized tissue prepared by conventional technologies, strength or the like is compromised, however it is depending on the case. Tissues , which have been decellularized to remove the cells therein, have shown in some cases where the strength thereof is reduced. Accordingly, it is an object of the present invention to provide a method for reinforcing tissue strength of such a decellularized tissue.

Many researchers recognize a number of utilities of decellularized tissues, including the physical and biological properties thereof. However, at present, decellularized tissues available have deficiencies such as calcification and elicit immunological reactions and the like, and is thus in need of further improvement (Christine E.Schmidt,Jennie M.Baier., 2000,Biomaterials 21:2215-2231).

(Means for Solving the Problems)

The present inventors have discovered that inclusion of biocompatible macromolecule into a decellularized tissue unexpectedly enhances the decellularized tissue, to solve the above-described problem. The present inventors have also discovered that the decellularized tissues may attain unexpectedly strong reinforcement effects by dimerizing such biocompatible macromolecules by means of radical reaction, preferably using gamma-ray. Decelluarization may be performed by any known technology in the art, and for example, a surfactant such as SDS or an amphipathic molecule such as PEG may be used.

Decelluarized tissues thus prepared are minimized with respect to damage to extracellular matrices. The decellularized tissue according to the present invention may be used, for example, as an artificial blood vessel which can utilized permanently. As such, it can be recognized that utilities of the decellularized tissue of the present invention extends to a vast range of applications.

Furthermore, the decellularized tissue reinforced by the present invention has a significant and unexpected improvement in the tissue strength. Such unexpected effects cannot be attained by conventional art. The provision of such reinforced decellularized tissues and tissue grafts leads to significant progress in transplantation medicine and the significance thereof should be of note.

Therefore, the present invention provides the following:

(1) A decellularized tissue comprising a biocompatible macromolecule .

(2) A decellularized tissue according to Item 1, wherein:

a) a cell redisual rate of the tissue is less than a level at which an immune reaction is elicited in an organism; and
b) the tissue is not damaged to such an extent that hinders the tissue from exhibiting a function which was possessed by the tissue when the tissue was not decellularized.

(3) A decellularized tissue according to Item 1, wherein the biocompatible macromolecule coates the tissue.

(4) A decellularized tissue according to Item 1, wherein the biocompatible macromolecule is crosslinked with the tissue.

(5) A decellularized tissue according to Item 1, wherein the biocompatible macromolecule is crosslinked with the tissue by means of a radical reaction.

(6) A decellularized tissue according to Item 1, wherein the biocompatible macromolecule is crosslinked with a irradiation selected from the group consisting of ultraviolet irradiation, exposure to a free radical source, ultrasonication, x-ray irradiation, gamma-ray irradiation and electron beam irradiation.

(7) A decellularized tissue according to Item 1, wherein the biocompatible macromolecule is biodegradable.

(8) A decellularized tissue according to Item 1, wherein the biocompatible macromolecule comprises a macromolecule selected from the group consisting of polyvinyl alcohol (PVA), polyvinyl pyrrolidone (PVP), elastin, polyethylene glycol (PEG), gelatin, collagen, gamma-polyglutaminic acid, and a mixture of the two or more thereof.

(9) A decellularized tissue according to Item 1, wherein the biocompatible macromolecule comprises polyvinyl alcohol or polyvinyl pyrrolidone.

(10) A decellularized tissue according to Item 1, wherein the polyvinyl alcohol is in the range of molecular weight of 500 to 200,000.

(11) A decellularized tissue according to Item 1, wherein the cell redisual rate of the tissue is 30% or less..

(12) A decellularized tissue according to Item 1, wherein the tissue damage rate of the tissue is 30% or less.

(13) A decellularized tissue according to Item 1, wherein the tissue has a tissue strength which permits a clinical application.

(14) A decellularized tissue according to item 1, wherein the tissue has a tissue strength which is 80% or more of a tissue strength which was possessed by the tissue when the tissue was not decellularized.

(15) A decellularized tissue according to item 1, wherein the tissue has a tissue strength having a $\beta$ value which is 80% or more of a $\beta$ value which was possessed by the tissue when the tissue was not decellularized.

(16) A decellularized tissue according to item 1, wherein the tissue has a tissue strength having a $\beta$ value of 20% or more.

(17) A decellularized tissue according to item 1, wherein the tissue is membranous, valvular, or luminal tissue.

(18) A decellularized tissue according to item 1, wherein the tissue is tissue selected from blood vessels, blood vessel-like tissue, cardiac valves, pericardia, dura mater, corneas, and bones.

(19) A decellularized tissue according to item 1, wherein a state of the tissue, in which the tissue is not damaged to such an extent that hinders the tissue from exhibiting a function which was possessed by the tissue when the tissue was not decellularized, includes that an extracellular matrix of the tissue is not substantially degenerated.

(20) A decellularized tissue according to item 18, wherein a survival rate of the extracellular matrix is at least about 50%.

(21) A decellularized tissue according to item 1, wherein the tissue is derived from a mammal.

(22) A decellularized tissue according to item 1, wherein the tissue is derived from a human or a swine.

(23) A tissue graft comprising a decellularized tissue according to Item 1.

(24) A tissue graft according to Item 23, further comprising a cell.

(25) A tissue graft according to Item 23, wherein the tissue graft is free of a cell.

(26) A tissue graft according to Item 23, wherein the tissue graft has a form of selected from the group consisting of membranous, valvular, or luminal form.

(27) A method of producing a decellularized tissue, comprising the steps of:

    1)providing tissue; and
    2)decellularizing the tissue; and
    3)exposing the tissue to a biocompatible macromolecule.

(28) A method according to Item 27, wherein the step of decellularizating comprises immersing the tissue in a solution containing a non-micellar amphipathic molecule or a solution containing a surfactant.

(29) A method according to Item27, wherein the step of exposing the tissue to a biocompatible macromolecule comprises crosslinking the biocompatible macromolecule.

(30) A method according to Item 29, wherein the crosslinking comprises a radical reaction.

(31) A method according to Item 29, wherein the radical reaction comprises a irradiation selected from the group consisting of ultraviolet irradiation, exposure to a free radical source, ultrasonication, x-ray irradiation, gamma-ray irradiation and electron beam irradiation.

(32) A method according to Item 29, wherein the radical reaction is gamma-ray irradiation.

(33) A method according to Item 32, wherein the irradiation dose of the gamma-ray irradiation is in the range of 10-300 kGy.

(34) A method according to Item 32, wherein the gamma-irradiation is conducted under a circumstance selected from the group consisting of in vacuum, in oxygen, in nitrogen, in the air, in water, in an amphipathic molecule solution and a combination thereof.

(35) A method according to Item 32, wherein the gamma-irradiation is conducted for between 0.5-240 hours.

(36) A method according to Item 27, wherein the biocompatible macromolecule is biodegradable.

(37) A method according to Item 27, wherein the biocompatible macromolecule comprises a macromolecule selected from the group consisting of polyvinyl alcohol (PVA), polyvinyl pyrrolidone (PVP), elastin, polyethylene glycol (PEG), gelatin, collagen, gamma-polyglutaminic acid, and a mixture of the two or more thereof.

(38) A method according to Item 27, wherein the biocompatible macromolecule comprises polyvinyl alcohol or polyvinyl pyrrolidone.

(39) A method according to Item 38, wherein the polyvinyl alcohol is in the range of molecular weight of 500 to 200,000.

(40) A method according to Item 27, wherein the biocompatible macromolecule is used at the concentration between 1 w/v% to 50 w/v%.

(41) A method according to Item 28 , wherein the amphipathic molecule is a 1,2-epoxide polymer.

(42) A method according to Item 28, wherein the amphipathic molecule is polyethylene glycol (PEG).

(43) A method according to Item 27, wherein the decellularization step is performed for 30min to 10 days..

(44) A method according to Item 27, wherein the amphipathic molecule is biocompatible.

(45) A method according to Item 27, wherein the tissue is a tissue selected from blood vessels, blood vessel-like tissue, cardiac valves, pericardia, dura mater, corneas, and bones.

(46) A method according to Item 27, wherein the tissue is derived from a mammal.

(47) A method according to Item 27, wherein the tissue is derived from a human or a swine.

(48) A method according to Item 27, further comprising the step of a chemical treatment, which is a nuclease treatment.

(49) A method according to Item 48, wherein the chemical treatment comprises a treatment by means of DNase.

(50) A decellularized tissue obtainable by a method according to Item 27.

(51) A method for regenerating a tissue, comprising the steps of:

a)providing a decellularized tissue comprising a biocompatible macromolecule into an organism; and
b)incubating the tissue within the organism for a time sufficient for the tissue to regenerate.

(52) A method according to item 51, further comprising providing a cell to the decellularized tissue.

(53) A method according to item 51, further comprising providing a physiologically active substance which induces cellular differentiation, to the organism.

(54) A method according to item 53, wherein the physiologically active substance is from the organism or from outside the organism.

(55) A method according to item 52, wherein the physiologically active substance is provided in a form of nucleic acid or polypeptide form.

(56) A method according to item 53, wherein the physiologically active substance is selected from the group consisting of HGF, VEGF, FGF, IGF, PDGF and EGF.

(57) A method according to item 51, wherein the tissue is a tissue selected from blood vessels, blood vessel-like tissue, cardiac valves, pericardia, dura mater, corneas, and bones.

(58) A method for producing a tissue graft, comprising the steps of:

a)providing a decellularized tissue comprising a biocompatible macromolecule into an organism;
b) allowing a self cell in the organism to infiltrate the decellularized tissue; and
c) incubating the tissue within the organism for a time sufficient for the cell to differentiate.

(59) A method according to Item 58, wherein the tissue is a tissue selected from blood vessels, blood vessel-like tissue, cardiac valves, pericardia, dura mater, corneas, and bones.

(60) A method according to Item 58, wherein the decellularization tissue is autologous.

(61) A method according to Item 58, wherein the decellularization tissue is derived from a homologous host.

(62) A method according to item 58, wherein the decellularization tissue is derived from a heterologous host.

(63) A method according to Item 58, further comprising the step of:

D) providing a physiologically active substance which induces differentiation of the cell.

(64) A method according to Item 63, wherein the physiological active substance is a cytokine having hemopoietic activity.

(65) A tissue graft produced by a method according to Item 58.

(66) A method of treating a subject requiring transplantation of tissue or an organ or treating a subject at a risk of transplantation of tissue or an organ for prophylaxis, the method comprising the steps of:

a)providing decellularized tissue comprising a biocompatible macromolecule or a tissue graft comprising the decellularized tissue into an organism; and
b) transplanting the decellularized tissue or tissue graft to a subject.

(67) A method according to item 66, wherein the tissue is derived from the subject.

(68) A method according to item 66, wherein the tissue is a tissue selected from the group consisting of blood vessels, blood vessel-like tissue, cardiac valves, pericardia, dura mater, corneas, and bones.

(69) A method according to item 66, wherein the subject is a mammal.

(70) A method according to item 66, wherein the subject is a human.

(71) A pharmaceutical for organ transplantation, comprising:

a)decellularized tissue comprising a biocompatible macromolecule or a tissue graft comprising the decellularized tissue into an organism.

(72) A pharmaceutical according to item 71, wherein the tissue is tissue selected from the group consisting of blood vessels, blood vessel-like tissue, cardiac valves, pericardia, dura matter, corneas, and bones.

(73) A pharmaceutical according to item 71, wherein the cell is derived from a mammal.

(74) A pharmaceutical according to item 71, wherein the tissue is derived from a human.

(75) A pharmaceutical according to item 71, wherein the tissue is derived from the subject requiring transplantation.

(76) Use of decellularized tissue comprising a biocompatible macromolecule or a tissue graft comprising the decellularized tissue for manufacture of a pharmaceutical for organ transplantation.

Accordingly, it is understood that these and other advantages of the present invention will be clear to those skilled in the art upon reading and understanding the following detailed description in view of the drawings attached hereto.

EFFECTS OF THE INVENTION

Decellularized tissue with the strength is reinforced, improved or maintained, is provided. Furthermore, the present invention attains effects of reinforcement of decellularized tissue, reduced immunological rejection reaction, or elasticity is improved.

BRIEF DESCRIPTION OF THE DRAWINGS

Figure 1 depicts a decellularized tissue of the present invention after treatment with a biocompatible molecule.

Figure 2 depicts the results of tensile test of a native canine aorta.

Figure 3 depicts the results of tensile test of an artificial valve (conventional artificial valve) prepared by the third decellularized method using SDS.

Figure 4 depicts the result of a tensile test of the decellularized tissue of the present invention with a coating of PVA onto an artificial valve prepared by the third decellularized method using SDS.

Figure 5 depicts another result of a tensile test of the decellularized tissue of the present invention with a coating of PVA onto an artificial valve prepared by the third decellularized method using SDS.

Figure 6 depicts results of tensile test of a decellularized valve and a PVA coating.

Figure 7 depicts the results of subcutaneous transplantation of a decellularized tissue of the present invention.

Figure 8 depicts the results of prosthetic treatment (evaluation by tensile strength) of a membrane treated with SDS-Triton X100.

Figure 9 depicts the results of prosthetic treatment (evaluation by rat dorsal transplantation) of a membrane treated with SDS and Triton X-100. Figure 9 shows (1) transplantation site: rat dorsal portion; (2) two weeks after the transplantation; (3) HE stain; and (4) tissue photographs taken at x100 magnification.

Figure 10 depicts uses a decellularized tissue prepared by decellularization treatment method No. (2) to confirm presence/absence of rejection reaction. Experimental conditions used in Figure 10 is gamma-ray 15kGy irradiation -> PEG -> DNase treatment for membrane prosthetic treatment (evaluation is made by means of rat dorsal transplantation).

Figure 11 depicts the results of gamma-ray 100kGy irradiation -> PEG -> DNase treatment for membrane prosthetic treatment (evaluation is made based on tension strength). In the figure, the results obtained by treatment using PVP are shown.

Figure 12 depicts the results of gamma-ray 100kGy irradiation -> PEG -> DNase treatment for membrane prosthetic treatment (evaluation is made based on elasticity). In the figure, the results obtained by treatment using PVP are shown.

Figure 13 depicts the results of gamma-ray 100kGy irradiation -> PEG -> DNase treatment for membrane prosthetic treatment (evaluation is made by means of rat dorsal transplantation). Results are shown for (1) transplantation site: rat dorsal site; (2) two weeks after the transplantation; (3) HE staining; and (4) magnification: x 100 for the experiments.

DETAILED DESCRIPTION OF THE INVENTION

It should be understood throughout the present specification that expression of a singular form includes the concept of their plurality unless otherwise mentioned. Specifically, articles for a singular form (e.g. , "a", "an", "the", etc. in English; "ein", "der", "das", "die", etc. and their inflections in German; "un", "une", "le", "la", etc. in French; "un", "una", "el", "la", etc. in Spanish, and articles, adjectives, etc. in other languages) include the concept of their plurality unless otherwise mentioned. It should be also understood that the terms as used herein have definitions typically used in the art unless otherwise mentioned. Thus, unless otherwise defined, all scientific and technical terms have the same meanings as those generally used by those skilled in the art to which the present invention pertain. If there is contradiction, the present specification (including the definition) precedes.

(Definition of Terms)

The definitions of terms used herein are described below.

(Regeneration Medicine)

As used herein, the term "regeneration" refers to a phenomenon in which when an individual organism loses a portion of tissue, the remaining tissue grows and recovers. The extent or manner of regeneration varies depending among animal species or among tissues in the same individual. Most human tissues have limited regeneration capability, and therefore, complete regeneration is not expected if a large portion of tissue is lost. In the case of heavy damage, tissue having strong proliferation capability, tissue different from that which is lost may grow resulting in incomplete regeneration where the damaged tissue is incompletely regenerated and the function of the tissue cannot be recovered. In this case, a structure made of a bio-absorbable material is used to prevent tissue having a strong proliferation capability from infiltrating the defective portion of the tissue, so as to secure space for proliferation of the damaged tissue. Further, by supplement with a cell growth factor, the regeneration capability of the damaged tissue is enhanced. For Example, such a regeneration technique is applied to cartilages, bones, and peripheral nerves. It has been so far believed that nerve cells and cardiac muscles have no or poor regeneration capability. Recently, it is of note that regeneration medicine is primarily using tissue grafts from self tissue.

As used herein, the term "cell" is defined as having the broadest meaning used in the art, referring to a structural unit of multi-cellular organisms, which has an enveloping membrane structure for separating the cell from the outside, has self-regeneration capability, and which is a living body having genetic information and an expression mechanism. In the method of the present invention, any cell can be used as a subject. The number of cells used in the present invention can be counted through an optical microscope. When counting using an optical microscope, the number of nuclei are counted. Tissues are sliced into tissue sections, which are then stained with hematoxylin-eosin (HE) to variegate nuclei derived from extracellular matrices (e.g., elastin or collagen) and cells. These tissue sections are observed under an optical microscope and the number of nuclei in a particular area (e.g. , 200 $\mu$m x 200 $\mu$m) can be estimated to be the number of cells.

Cells may elicit calcification and immune reactions. Therefore, non-self cells should be removed as much as possible for implantation of tissue or organs. In the case of self cells, decellularization is not required, since no immunological rejection problem is usually raised. However, since decellularization is sometimes preferable, cells should also be removed as much as possible. There is a strong desire for decellularized tissue to be provided. On the other hand, cells support the strength of a tissue, and thus simple decellularization may cause problems in terms of strength.

As used herein, the term "cell replacement" indicates that cells originally existing are replaced with other infiltrating cells in decellularized tissue. This term is also referred to as "cellular infiltration". Preferably, in the.present invention, cell replacement is carried out with cells of a host undergoing implantation.

As used herein, the term "tissue" refers to a group of cells having the same function and form in cellular organisms. In multi-cellular organisms, constituent cells are usually differentiated so that the cells have specialized functions, which results in division of labor. Therefore, multi-cellular organisms are not simple cell aggregations, but constitute organic or social cell groups having a certain functions and structures. Examples of tissue include, but are not limited to, integument tissue, connective tissue, muscular tissue, nervous tissue, and the like. Tissue targeted by the present invention may be derived from any organ or part of an organism. In a preferred embodiment of the present invention, tissue targeted by the present invention includes but is not limited to, blood vessels, blood vessel-like tissue, cardiac valves, pericardia, dura matter, corneas, and bone tissue. Since non-micellar amphipathic molecules used in the present invention acts in a mechanism like cell component extraction, principally tissue derived from any organ can be treated with the method of the present invention. Accordingly, it is understood that the treatment used in the biocompatible molecule of the present application can be targeted to any type of tissue.

As used herein, "membrane-like tissue" is also referred to as "planar tissue" and refers to a tissue having a membrane form. Membrane-like tissue includes tissue from organs, such as pericardia, dura matter, and corneas.

As used herein, "organ" or "part" is used interchangeably, referring to a structure which is a specific portion of an individual organism where a certain function of the individual organism is locally performed and which is morphologically independent. Generally, in multi-cellular organisms (e.g., animals and plants), organs are made of several tissues in a specific spatial arrangement and the tissues are made of a number of cells. Examples of organs or parts include organs or parts related to a blood vessel system. In one embodiment, examples of organs targeted by the present invention include ischemic organs (the heart undergoing cardiac infarction, skeletal muscle undergoing ischemia, and the like). In one preferred embodiment, organs targeted by the present invention are blood vessels, blood vessel-like tissue, cardiac valves, pericardia, dura matter, corneas, and bones. In another preferred embodiment, organs targeted by the present invention are cardiac valves, pericardia, and blood vessels.

In order to prepare grafts suitable for implantation, it may be optionally desirable that the organs are cultured. Organ culture refers to *in vitro* culture of the whole or part of an embryonic or mature organ extracted from an or ganism where the structure, function and differentiation thereof are maintained. In contrast, general tissue culture mainly aims at cell proliferation, where de-differentiation is likely to occur. Organ culture is conducted by a method including but not limited to, the watch glass method, the block Petri dish method, the support method, the sponge matrix method, the Trowell method, and the like. Also, large-scale cell culture (e.g., the hollow fiber method, the fixed bed method, the spin filter method, the precipitation tank method, perfusion culture, circulation type organ culture, etc. as described in Tanpakushitsu · Kakusan · Koso [Protein · Nucleic acid Enzyme] 45, 2188-2200(2000), may be used. An organ or tissue prepared by the decellularization method of the present invention is placed in a culture medium, which is supplemented with various substances so as to regulate the structure, function, growth and differentiation, organ-to-organ interaction, and the like.

As used herein, the term "decellularization" and "decellularize" refers to the removal of cells from tissues or organs. Preferably, decellularization may be carried out without damage to the structure and function of the original tissue or organs. Plasma components, cytosol components, cytoskeletons, and cell membrane components are removed from decellularized tissue. However, components required for maintaining the structure of tissue, such as extracellular matrix (ECM) components (e.g., elastin, collagen (Type I, IV), laminin, and the like), are maintained without degeneration. Therefore, decellularized tissue or organs are preferably substantially equivalent to an untreated tissue or organ in terms of properties thereof , such as shape, physical strength, elasticity, flexibility, and the like. Since extracellular matrices are not degenerated after implantation, it is preferable to provide an environment suitable for the infiltration, adhesion, proliferation, and expression and maintenance of differentiation traits, of cells of recipients, so that the matrices are replaced with a tissue made of self cells. The extent of decellularization can be measured using a cell redisual rate or survival ratio as an index.

In the present invention, the decellularization is performed using either a surfactant or amphipathic molecule, preferably a amphipathic molecule is used.

MHC class I and II proteins are preferably removed from the decellularized tissue. MHC class I and II proteins can be confirmed by SDS PAGE and/or western blotting analysis. Other extracellular matrix proteins can also be confirmed by SDS PAGE and/or western blotting analysis. Structural proteins in cells (collagen, elastin, and the like) can be evaluated by amino acid analysis (e.g., Edman degradation, automatic analysis using a peptide analyzer available from PE Biosystems, or the like). As a result, the influence of decellularization processes on ECM can be determined. Lipids and phospholipids contained in the cell membrane and cells can be analyzed using thin layer chromatography and HPLC. Sugar chains (e.g., glycosaminoglycans or the like) can be analyzed by agarose gel electrophoresis or the like. This analysis can analyze the glycosaminoglycan composition of extracellular matrices as well as the presence of $\alpha$-Gal or the like.

As used herein, "cell redisual rate (or ratio)" refers to a ratio of survival cells after decellularization of tissue by the method of the present invention to cells originally existing in the tissue. As used herein, the cell redisual rate is typically measured by a counting method using a microscope after hematoxylin and eosin staining (H&E staining). This method comprises counting nuclei variegated by HE staining under an optical microscope. Therefore, for example, this method comprises the following steps: variegating samples by HE staining; counting the number of nuclei (the number of cells) present in an area of 100mm x 100mm in the sample; optionally repeating the counting step (e.g., a total of 8 times) and averaging the counts; and calculating a ratio to a control (e. g. , untreated tissue is regarded as 100%). The ratio of the control (e.g.. a ratio of survival nuclei to untreated tissue) can be regarded as a cell redisual rate. Therefore, in this case, the cell redisual rate (%) = (the number of nuclei in treated tissue)/(the number of nuclei in untreated tissue) x 100. The cell redisual rate can also be determined by measuring the amount of survival DNA. This is because the total amount of DNA in cells of tissue is known to be generally proportional to the number of the cells of the tissue" Methods for measuring DNA are known in the art. For example, the amount of DNA extracted from tissue can be determined using a fluorescent reagent, such as Hoechst 33258 (from Molecular Probes) or the like. Specifically, tissue is isolated by ultrasonic pulverization treatment or the like; and the amount of DNA in extract supernatant can be determined by reacting with Hoechst 33258 (Ex (Excitation wavelength) 356, EM (Emission wavelength) 492) capable of specifically binding to DNA components in buffer solution and emitting fluorescence, and measuring the strength of fluorescence.

The term "less than a level which can elicit an immune reaction in organisms" of the cell redisual rate of certain tissue indicates that when the tissue is implanted into an organism, no immune reaction is elicited for a certain period of time (e.g., several months to several years, preferably the entirety of the rest of life).

As used herein, the term "immune reaction" refers to a reaction due to loss of coordination of immunologic tolerance between a graft and a host, including for example, hyperacute rejection reactions (within several minutes after implantation; immune reactions due to an antibody, such as $\beta$-Gal or the like), acute rejection reactions (cell-mediated immune reactions about 7 to 21 days after implantation), chronic rejection reactions (rejection reactions due to cell-mediated immune response after three months), and the like.

As used herein, elicitation of immune reactions can be determined by histological examination of the type, number, or the like, of cells (immune cells) infiltrating the implanted tissue by observing under a microscope, tissue sections stained by hematoxylin (HE) staining or immunological staining.

As used herein, the term "calcification" refers to precipitation of calcareous substances in organisms.

As used herein, "calcification" *in vivo* can be determined by measuring calcium concentration. Specifically, implanted tissue is taken out; the tissue section is dissolved by acid treatment or the like; and the atomic absorption of the solution is measured by a trace element quantifying device.

As used herein, the term "within organism(s) (or in organism(s))" or *"in vivo"* refers to the inner part of an organism(s). In a specific context, "within organism(s)" refers to a position at which a subject tissue or organ is placed.

As used herein, "*in vitro*" indicates that a portion of an organism is extracted or released outside the organism for various purposes of research (e.g., in a test tube). The term *in vitro* is in contrast to the term *in vivo.*

As used herein, the term "*ex vivo*" refers to a series of operations where target cells into which a gene will be introduced are extracted from a subject. A therapeutic gene is introduced *in vitro* into the cells, and the cells are returned into the same subject.

As used herein, the term "a function in the untreated (or naive) form" of certain tissue refers to a function possessed *in vivo* by the tissue in its normal state, For example, in the case of cardiac valves, cardiac valves usually have a function of preventing the back flow of blood from a ventricle to an atrium or from the pulmonary artery and aorta to an atrium, a function in the untreated form of cardiac valves refers to the function of preventing a back flow of blood from a ventricle to an atrium or from the pulmonary artery and aorta to an atrium.

As used herein, the term "extracellular matrix" (ECM) refers to a substance existing between somatic cells, no matter whether the cells are epithelial cells or non-epithelial cells. Extracellular matrices are involved in supporting the tissue as well as the internal environmental structure that is essential for survival of all somatic cells. Extracellular matrices are generally produced from connective tissue cells. Some extracellular matrices are secreted from cells possessing basal membrane, such as epithelial cells or endothelial cells. Extracellular matrices are roughly divided into fibrous components and matrices filling there between. Fibrous components include collagen fibers and elastic fibers. A basic component of matrices are a glycosaminoglycan (acidic mucopolypolysaccharide), most of which is bound to a non-collagenous protein to form a polymer of a proteoglycan (acidic mucopolysaccharide-protein complex). In addition, extracellular matrices include glycoproteins, such as laminin of the basal membrane, microfibril around elastic fibers, fibronectins on cell surface, and the like. Particularly differentiated tissue has the same basic structure. For example, in hyaline cartilage, chondroblasts characteristically produce a large amount of cartilage matrices including proteoglycans. In bones, osteoblasts produce bone matrices which cause calcification. One embodiment of the present invention may be characterized in that extracellular matrices (e.g., elastin, collagen (e.g., Type I, IV, and the like), laminin and the like) are not substantially changed before the decellularization treatment of the present invention.

As used herein, the term "tissue damage rate (or ratio)" refers to a parameter indicating a function of a tissue or organ, which is an index of how much a tissue or organ is damaged before and damaged after treatment, or an index of how much an original function of the tissue or organ can be exhibited. As used herein, a method for measuring a tissue damage rate is well known in the art. For example, the rate can be determined by counting elastin rupture sites. In a method used herein, a visual field is divided into units of $100\mu m \times 100\mu m$ and the number of units having an elastin rupture site are counted. There were 24 units per visual field. In HE-stained tissue sections, extracellular matrices were counted under a microscope. A damage rate is calculated as x/24, where untreated tissue is assumed to have a damage rate of 0%, i.e., x=0.

As used herein, the "tissue strength" refers to a parameter indicating a function of tissue or an organ, which is the physical strength of the tissue or organ that is generally determined by measuring tensile strength. A general tensile testing technique is well known in the art.

In the present specification, rrupture load is employed as strength, which is measured as follows : a specimen is cut out into a strip of 5mm in width and 30mm in length (basically aortic base portion is cut such that the wall portion thereof is longer in the direction of the length);

> (2) the specimen is fixed for about 5min in the fixing member of tension testing machine (universal testing apparatus TENSILON RTC1150A is used: available from ORIENTEC); and
>
> (3) the specimen is stretched at a test speed of 1 cm/min by the tension testing machine to indicate a point of rupture load. Herein, rupture load and elastic module can be measured.

The tissue strength of the decellularized tissue of the present invention in terms of the maximum load thereof may be usually at least about 8N, preferably at least about 10N, and more preferably at least about 11N. In the prior decellularized tissue or naturally-occurring tissue (for example, arterial), the strength thereof is usually about 7N. Further, conventional decellularization treatments have little effects on enhancement of the tissue strength. As such, the capability of tissue strength enhancement by the present invention is unexpectedly significant.

In a preferable embodiment, in terms of the coefficient of elasticity, the decellularized tissue of the present invention usually has a coefficient of elasticity of at least about 1.2MPa or greater, preferably at least about 1.6MPa. The decellularized tissue of the present invention may have a coefficient of elasticity less that that of a naturally occurring tissue (for example, 0.8MPa or greater, or 0.8-1.0MPa or the like), as long as the decellularized tissue can be used for the purpose of the present invention.

In measuring extension, the tensile testing apparatus used in the strength measurement test (TENSILLON ORIENTEC) may be used. Specifically, rectangular material having a width of 5mm and a length of 30mm is loaded with weight at a speed of 10mm/min, and thus rupture load and elasticity efficiency may be measured. Specifically, measurement of extension may be obtained by measuring the length of each direction before and after the tension stimulus, and by dividing the length after the tension by that prior to the tension and multiplying the result. Usually, the extension is measured for both longitude and horizontal directions. The extension for both directions is preferably uniform but it is no limited thereto. Depending on the application thereof, the properties relating to the extension may be but is not limited to, e.g. at least 120%, preferably 150 %. With respect to the extension properties, the decellularized tissue of the present invention may have extension less than that possessed by a native tissue (for example at least 50 % or the like), as long as it can be used.

By analyzing data obtained by a general tensile testing method, various data such as breaking strength, stiffness, Young's modulus, and the like can be obtained and can be herein and used as indexes of tissue strength. As used herein, in the case of luminal tissue, tissue strength can be represented by a stiffness parameter (β value). A β value is calculated by the following formula after the P-D (pressure-diameter) relationship is prepared:

$$Ln(P/Ps) = \beta(D/Ds-1) \qquad (1)$$

where Ps and Ds represent standard values at 100mmHg and P and D represent pressure and diameter, respectively.

The opposite ends of luminal tissue, such as a blood vessel or the like, are fixed to pipe-like units or the like and the outside and inside of the luminal tissue are filled with physiological saline. In this situation, pressure is applied to the inside of the tissue by an external device, while monitoring the outer diameter of the tissue. The measured pressure and diameter are inserted into formula (1) to obtain a β value (Sonoda H., Takamizawa K., et al., J. Biomed. Mater. Res. 2001:266-276).

As used herein, the term "amphipathic molecule" refers to a molecule having both a hydrophilic group (carboxy group, sulfate group, quaternary ammonium group, hydroxy group, etc.) and a hydrophobic group (also referred to as lipophilic group; including a long chain hydrocarbon group and the like). Amphipathic molecules have affinity for either polar solvents or non-polar solvents. This property is called amphiphilicity.

As used herein, the term "surfactant" refers to a substance which is dissolved in a liquid and has an action to significantly reduce the surface tension of the liquid. There is a hydrophilic portion and a hydrophobic portion separately present within the molecule, so that the molecule is easily adsorbed onto a surf ace. Surfactant molecules form a molecular cluster, which is called a micelle, at a certain concentration (critical micelle concentration) or more. Therefore, the term "surfactant" as used herein partially overlaps the term "amphipathic molecule". As used herein, the term "micelle" refers to a cluster of amphipathic molecules, such as a surfactant, formed when the molecules are dissolved in water at a certain concentration or more, in which the hydrophilic groups face the outside of the cluster while the lipophilic groups face the inside of the cluster. Formation of micelles abruptly occurs at a certain concentration, which concentration is called "critical micelle concentration". A property of an aqueous solution significantly changes at this concentration. Micelle-forming molecules are strongly absorbed onto a two-phase interface due to the hydrophilic-lipophilic balance of a surfactant. As a result, the free energy of the interface is significantly reduced so that cell components, such as proteins, lipids, and the like, are solubilized. Surfactants for decellularization are generally used at a critical micelle concentration or more (e.g., 1%). Therefore, decellularization treatment is achieved by a mechanism of decreasing free energy. A critical micelle concentration is determined depending on the substance, and is well known or can be determined by preparing its aqueous solution.

As used herein, the term "non-micelle forming" refers to a property of an amphipathic molecule which does not form a micelle at a predetermined concentration. Preferably, the property of forming no micelles may be maintained at any concentration. It is desirable that substances used in the present invention (e.g., polyethylene glycol) have no critical micelle concentration in an aqueous solution.

As used herein, the term "non-micelle forming amphipathic molecule" refers to an amphipathic molecule which does not form micelles at a predetermined concentration. Preferably, such a molecule has no critical micelle concentration within a concentration range, in which such a molecule can exist as an aqueous solution, can be dissolved in water. Therefore, the molecule is not micellar at any concentration. When a non-micelle forming amphipathic molecule is used, decellularization treatment may be carried out by a mechanism similar to extraction of cell components using a chemical extraction technique. Therefore, decellularization treatment using a non-micelle forming amphipathic molecule solution is different from conventional decellularization treatment using a surfactant. As a result, a significant difference occur s in the degree of tissue damage. For decellularization treatment, any non-micelle forming amphipathic molecule, preferably 1,2-epoxide polymer, and more preferably polyethylene glycol, may be herein used.

As used herein, the term "non-micellar" refers to a state of not forming micelles when an amphipathic molecule is mentioned. Such a non-micellar state may be achieved by an amphipathic molecule at less than a critical micelle concentration or a non-micelle forming amphipathic molecule. Therefore, such a non-micellar state may be achieved with 1,2-epoxide polymer (particularly, polyethylene glycol). Alternatively, a surfactant, such as SDS, Triton X-100 (registered trademark), or the like, may be used to achieve a non-micellar state when the surfactant is present at less than a critical micelle concentration, which can be used in the present invention.

As used herein, the term "1,2-epoxide polymer "refers to a polymer formed by polymerization of 1,2-epoxide as a monomer. 1,2-epoxide polymers include, but are not limited to, ethylene oxide polymers or copolymers therewith, propylene oxide polymers or copolymers therewith, and higher 1,2-epoxide polymers or copolymers therewith. 1, 2-epoxide is a compound having a structure in which oxygen atoms are bound to two carbon atoms which has been bound to each other within the molecule, i.e. , an oxygen atom is bound to each of positions 1 and 2. Examples of 1,2-epoxides include, but are not limited to, ethylene oxide, propylene oxide, butylene oxide, epichlorohydrin, and the like. Examples of 1,2-epoxide polymers include, but are not limited to, ethylene oxide polymers, propylene oxide polymers, copolymers therewith, polypropylene glycol, polyethylene glycol, and the like. Preferably, 1,2-epoxide polymers include, but are not limited to, polyethylene glycol, polypropylene glycol, and the like. A more preferable 1,2-epoxide polymer is polyethylene glycol. Preferably, 1,2-epoxide polymers have amphipathicity.

As used herein, the term "polyethylene glycol (PEG)" refers to a polymer of ethylene glycol, and is also referred to as polyethylene oxide (poly(ethylene oxide)) represented by $HO-(CH_2CH_2O)_n-H$. Polyethylene glycol is commercially available from various companies, such as, for example, Union Carbide (Carbowax), Dow (Polyglycol E), Texaco Chemical (Jeffox), Olin (PolyG), BASF Wyandotte (Pluracol E), Hodag, ICI Americas (ATEG), Nacalai tesque, NOF Corporation, and the like. Typically, PEG has an average molecular weight of between 200 and 6000. Such a PEG is, for example, polyethylene glycol ($H(OCH_2CH_2)_nOH$) having a # molecular weight of, for example, 200, 600, 1000, 2000 or 6000 from Nacalai tesque. Preferably, PEG has an average molecular weight of between 1000 and 2000. In another preferred embodiment, PEG may have an average molecular weight of 1000 or less. More preferably, PEG may have an average molecular weight of between 1500 and 2000. The above-described PEGs are commercially available and can be appropriately synthesized in order to achieve the desired characteristics. Such a synthesis method is well known in the art . Note that average molecular weights and molecular weights are herein represented by Daltons. Preferably, PEG used in the present invention has a uniform molecular weight. This is not essential. PEG having an average molecular weight within a certain range can usually be used. In the present invention, a substance (e.g., an enzyme (e.g., DNaseI or the like), an enzyme inhibitor, or the like) can be added in addition to PEG for the purpose of decellularization. When a chemical substance is added in addition to PEG, the amount or concentration of PEG to be added may vary depending on the amount of the existing chemical substance. A concentration used for cell fusion or the like is preferable. For example, a concentration of 1g/ml or more (100w/wormore) is preferable, but the present invention is not limited to this. Alternatively, PEG may be used at the concentration of 60%w/v to 100%w/v. Polyethylene glycol may also be used in a method for reinforcing a decellularized tissue after the tissue has been prepared via methods of the present invention.

As used herein the term "biocompatibility" refers to properties which allows survival without disorders *in vivo* due to lack of toxicity or immunological rejection capability.

As used herein the term"biocompatible macromolecule" refers to a macromolecule having good biocompatibility. Specifically, macromolecule does not cause toxicity even if it remains in the body. A method for judging whether a certain macromolecule has biocompatibility or not, uses a test method such as enthesis experiments under the skin of an experimental animal such as a rat or the like. In the present experimental method, as a result of subcutaneous enthesis experiments, relatively acute immunological reactiions or allergic reactions and the like will occur, also an enlarged, rubefied and fevered, in which case it is grossly understood that biocompatibility is low. Furthermore, in the cases where an artificial vessel is transplanted into a specific diseased portion, such as the cases when an artifical vessel is transplanted into a vessel of an animal, or the like, transplanted sites are observed several days to several months after the transplantation to observe the presence or absence of fixed tissues, the extent of inflammation, fusion,thrombus formation due to blood coagulation and the like , so as to determin tissue compatibility. In addition, hematoxylin/eosin staining or the like is made to the produced tissue section at the transplantation site to stain and observe the cells, and determination is made whether or not a number of granular cells responsible for immune system as an index for low tissue compatibility are invaded, or whether or not scar tissue formation separating both between conventional tissue and transplantation tissue. Furthermore, in particular as an index of high tissue compatibility of a decellularized tissue, it is determined by observing whether or not a variety of cells such as vascular endothelial cells, fibroblasts, smooth muscle cells and the like are invaded, and thus whether or not recellularization occurs (that is, transplantation tissue is fixed so as not to be distinguishable between the conventional tissue and transplantation tissue).

Furthermore, in addition to the determination by observing forms of tissues and cells as described above, cytokine concentration in the blood due to the progress of an inflammation reaction, antibody concentration (titer) against transplantation tissue recognized to be an foreign matter, concentratino of complement component, enzyme amount of drug metabolism enzyme (e.g. P450 and the like) induced by forein matter invasion, and the like (i.e. biochemical values) and the change in the values before and after the transplantation, which can be indices for tissue compatibility.

With respect tomaterial used for the medical apparatus, in order to rationally regulate the use thereof for medical apparatus by evaluating the toxicity thereof, there are a number of test items such as cell toxicity test, sensitization test, stimulus test, enthesis test, genetic toxicity test, blood compatibility test, systemic toxicity test and the like, which are regulated in the Guidelines by the Japan Ministry of Welfare, Labour and Health, the US National Standards, and individial test methods defined by international industrial standard such as ISO-10993 and the like.

Biocompatible macromolecules include, but are not limited to, for example, polyvinyl alcohol, poly lactic acid, polyglycolic acid, polyvinyl pyrrolidone, polyethylene glycol, gelatin, collagen, gamma-polyglutamic acid, silicone, polyvinyl chloride, polymethacrylate methyl, polytetrafluoroethylene, polyester, polypropylene, polyurethane, cellulose, polystyrene, nylon, polycarbonate, pyrisulfone, polyacrylonitrile, colalgen, gelatin, fibrin, chitin, chitosan, alginate, starch, gamma-polyglutamic acid, polycaprolactone, polyhydroxy butyrate, and the like. Among the polyvinyl alcohol, in addition to those unmodified, there are also those with chemical modifications such as modifications to the amino group, carboxy group, acetyl group and the like, which are also commercially available. These modified polyvinyl alcohols may also be used in the present invention. Preferably, biocompatible macromolecules are biodegradable but are not limited thereto.

As used herein, the term "polyvinyl alcohol" is also referred to as poval or PVA to refer to a macromolecule obtained by hydrolysis (more correctly ester exchange) of a polyacetic vinyl. It is expressed as a general formula $-(CH_2C(OH)H)_n-$. Vinyl alcohol cannot exist as a monomer, and thus can be produced by means of hydrolysis of polyvinyl acetate. It is primarily used for cell fusion.

As used herein the term "polyvinyl pyrrolidon" refers to a water soluble macromolecule compound set forth as a general formula $[-CH_2CH(C_4H_6NO)-]_n$, which are mainly used as an excipient for a drug.

As used herein the term "biodegradable" refers to properties, when referred to a material, in which it is degraded *in vivo* or by the action of microorganisms. Biodegradable macromolecules may be degraded into, for example, water, carbon dioxide, methane and the like by means of hydrolysis. As used herein, a method for determining whether biodegradation occurs, can be performed with respect to bioabsoptive property, a part thereof, by using an enthesis test for days to years into an experimental animal such as a rat, rabbit, dog and the like, with respect to degradation by microorganisms, embedded or enthesis test for days to years in the ground for macromolecules in a form of sheet, and enthesis and degradation tests and the like. With respect to transplantation, tests using an animal is preferable.

Furthermore, simpler alternative methods corresponding to the above-mentioned test, include a method of dissolution in a water-soluble solution of a variety of buffer such as phosphate buffered saline (PBS) for non-enzymatic macromolecule degradation, hydrolysis enzyme for the macromolecule for the enzymatic degradation of the macromolecule (protease, glycosidase, lipase, esterase and the like) are added to the buffer, which can be used. Biocompatible macromolecules include, but are not limited to, native and synthetic macromolecules. Native macromolecules include, for example, proteins such as collagen, starch, and the like, polysaccharides and the like, and synthetic macromolecules include, for example, fatty acid polyesters such as polyglycolic acid, polylactic acid, polyethylene succinate and the like. POlyvinyl alcohol exhibit weak biodegradative property with respect to biodegradative property. Therefore, as used herein PVA is recognized to have biodegradable property.

As used herein the term "macromolecule" is used interchangeably with "molecule" and is not limited to a specific molecular weight. If a macromolecule is discussed with a limitation of moleceular weight, it usually refers to those having molecular weight of 500 or greater but is not limited thereto. The upper limit of the molecular weight of the macromolecule used in the present invention may be infinite in principle. However, in the present invention, usually, it is discussable and handlable as a solution up to the molecular weight of 5,000,000 (in terms of active light scattering, gel filtration, sedimentation equilibrium by means of centrifugation apparatus, which are used for measuring molecular weight). As used herein, the term "biomacromolecule", "biocompatible macromolecule" are used, in a similar manner as in the art and field, to also refer to "biomaterial", "medical macromolecule" and the like as its synonym.

As used herein the term "immerse" refers to action of placing matter into a fluid (for example, liquid). In the present invention, "immersion" or "immerse" refers to placing a tissue to be treated in a solution containing a surfactant or an amphipatic molecule in a non-micelle state (e.g. 1,2-epoxide polymer such as polyethylene glycol). Accordingly, the step of immersion is preferably performed by completely immersing the tissue to be treated into the solution for subsequent treatment. Furthermore, in the step of immersion, it is preferable that physical treatment may be performed (e.g. rubbing with glass bar) in order to efficiently conduct removal of the components to be removed.

As used herein the term "expose" or "subject" refers to that a matter is in contact with an agent (for example, biocompatible macromolecule).

As used herein, the term "washing" step indicates that after performing the step of immersing tissue to be treated in a solution of a non-micellar amphipathic molecule (e.g., a 1,2-epoxide polymer) of the present invention, the tissue is removed from the tissue. Therefore, preferably, the washing step is performed using liquid. In the present invention, since treated tissue is intended to be used in an organism, the tissue is preferably washed with physiologically acceptable liquid. In one preferred embodiment, the washing step may be performed using PBS (phosphate buffered saline). A wash solution may optionally contain other pharmaceutical agents (e.g., a protease inhibitor). The other pharmaceutical agents are preferably not toxic and are biocompatible.

As used herein, the term "chemical treatment" refers to treating (e.g., immersing) a certain object with a chemical substance in a broad sense. Note that as used herein, the term "chemical treatment" refers to steps other than the step of immersing an object in a solution containing a 1,2-epoxide polymer (e.g., polyethylene glycol) as an essential step. For example, in a method of the present invention, when chemical treatment is performed in addition to the step of immersing an object in a solution containing polyethylene glycol having an average molecular weight of 1000 to 2000, chemical treatment includes the step of immersing the object in a solution other than the solution containing polyethylene glycol having an average molecular weight of 1000 to 2000 (e.g., a DNaseI solution, a glutaraldehyde solution, a solution containing polyethylene glycol having another average molecular weight, other 1, 2-epoxide polymer solutions, and the like, but not limited to these solutions).

As used herein the term "reinforce" refers to, when referring to tissues, that the tissue strength thereof is improved. Preferably, the tissue strength is preferably greater than originally possessed by the such as 110% or more, 120% or more, 150% or more, 200% or more as compared to that before the re-enforcement treatment. For example, such tissue strength may be expressed as maximum point load (for example, unit N) in the tension strength test in the claimed invention.

As used herein, the term "radical reaction" is also called group/radical leaving reaction and refers to a chemical reaction in which a free radical group is involved in the course of a reaction.

Radical reactions include, for example, as follows:

1) a reaction by cleavage of stabile bonds in a molecule to result in a free radical. e.g. $C_3H_8 -> CH_3 \cdot + C_2H_5 \cdot$

2) a reaction between a free radical and a stabile molecule to result in a different molecule and a different free radical. e.g. $CH_3 \cdot + CH_3COCH_3 -> CH_4 + CH_3COCH_3 \cdot$

3) a reaction between a free radical and a stabile molecule to form a larger free radical e.g. $CH_3 \cdot + C_2H_4 -> C_3H_7 \cdot$

4) a reaction of degradation of a free radical to result in a smaller free radical and a molecule

e.g. $n-C_3H_7 \cdot -> C_2H_4 + CH_3 \cdot$

5) a reaction between free radicals, which include:

recombination reaction $CH_3 \cdot + CH_3 \cdot -> C_2H_6$

disproportionation $C_2H_5 + C_2H_5 -> C_2H_4 + C_2H_6 \cdot$

As used herein the term "radical", also referred as "free radical", refers to a chemical species having an unpaired electron. Those having two unpaired electrons are called bi-radicals. Radicals include, but are not limited to, e.g., stable radicals, which can be handled as a normal substance, such as ozone, hydrogen peroxide, and 2,2-diphenyl-1-picrylhydradyl (DPPH), di-t-butyl aminoxyl and the like; radicals which can only exist in a solution such as triphenylmethyl free radical and the like.

As used herein, the term "free radical source" refers to an agent which can provide a radical, and includes, but is not limited to, a subtance in a radical state as described above, and apparatuses which are capable of providing a such a radical (for example, X-ray irradiation apparatus, ultraviolet irradiation apparatus, electron beam irradiation apparatus, gamma-ray irradiation apparatus, and the like), and the like.

Generally, such a free radical is produced by cleaving a bound chemical by means of, e.g., X-ray irradiation, ultraviolet irradiation, electron beam irradiation, gamma-ray irradiation; heat degradation, light degradation and radioative irradiation, degradation of a molecule, and donation and reception of an electron and the like. Free radicals thus produced are extremely rich in chemical activity such as methyl free radical $CH_3$ methylene free radical $:CH_2$ and the like, and rapid changes by reactions between radicals *per se,* or with a stable molecule (radical reaction). Such a free radical is an example of a free radical source used in the present specification.

Searching for such a product allows one to know whether or not and which free radical is produced and is reactive. Radicals may be confirmed by means of the progress in measureing methods such as chemical methods, electron spectrum or paramagnetic resonance absorption and the like. Radicals having extremely short life time (for example, OH) may be confirmed for its presence by means of flash photolysis method, rigid solvent method, and matrix isolation method and the like. When a metal atom of a transition metal complex has one or a plurality of unpaired electrons, it is not called a free radical.

As used herein the term "gamma-ray" refers to a electromagnetic wave having wavelength of shorter than 0.001 nm. When expressed in terms of energy, it corresponds to a light quantum of several hundreds of keV. Usually, gamma rays are released upon transition between energy levels of an atomic nucleus. The release source of gamma rays includes, for example, any radioactive source, and preferably a irradiation source of $^{60}$Co(cobalt60), $^{137}$Cs(cesium 137), as they are preferable for treating decellularized tissues.

Exposure or irradiation dose of gamma-rays is often expressed in kGy units. Exposure dose may be measured by measuring the absorbed dose. For such measurement, measuring devices such as cavity ionization chamber, calorimeter, film dosimeter, PMMA dosimeter and the like are suitable to be used.

As used herein the term "ultraviolet (ray or irradiation)" means an electromagnetic wave of wavelength within the range of about 360-400 nm as an upper limit for short wavelength of visible light and about 1 nm as a lower limit. The lower limit is not clear, and overlaps with soft x-rays for the range of tens of nm, and thus as used herein it is understood that ultraviolet ray refers to the range overlapping with x-ray. Light sources used therefor include but are not limited to, for example, quartz lamp, carbon arc lamp, spark discharge, hydrogen or raze gas discharge, synchrotron irradiation and the like.

As used herein the term "x-ray", also refers to roentgen, which refers to an electromagnetic wave having wavelength of 0.001 nm or greater, preferably 0.01 nm to tens of nm. X-rays have greater permeability in shorter wavelength, and gradually transits to gamma-ray, and has weaker permeability in longer wavelengths, and gradually transits to ultraviolet ray. Accordingly, the wavelengths of x-rays partially overlaps with those of gamma-rays and x-rays. Electrons emitted from heat negative poles of the X-ray tube is accelerated under high pressure, and collapsed with anti-negative poles (i.e. positive pole) which was cooled by water to form the x-ray, and is regardless of the material consisting of the anti-negative pole classified into two types: continuous x-rays of continuous spectra caused by controlled irradiation and specific x-rays having a line spectrum, which is inherent to the material of the anti-negative pole. Any type of x-ray may be used in the present invention. A irradiation source of x-rays usually include, an x-ray tube, including but not limited to heat electron x-ray tube (Coolige tube), gas x-ray tube and the like. An Electron beam emitted from the heat negative pole is focussed on the positive pole surface by focussing at Wehnelt electrode to cause x-rays. Windows from which x-rays axe extracted include beryllumplate and the like. Materials used for the positive pole include tungsten if continuous x-ray is permissible, and if one wishes to obtain specific X-ray in particular, Ka line, include iron, cuppex, molybdenium, silver and the like. Acceleration voltage is usually between the range of 30-100kV, but are not limited thereto. If one wished to obtain an x-ray having greater permeability, a higher voltage is used. As used herein, it is understood that any acceleration voltage may be used.

As used herein, radical reactions are usually achieved by exposure to a free radical but is not limited thereto. For example, radical reactions can be achieved by gamma-ray irradiation, ultraviolet irradiation, exposure to a free radical source, ultrasonication and x-ray and the like. Free radical sources include, for example, stable radicals as described above, radicals present in a solution and the like but are no limited thereto. Radical reactions by irradiation is preferable. This is because it can prevail the radical reaction effects to the entire tissue.

As used herein, the term "electron beam" refers to a beam of an electron having some substantially stable kinetic energy and has wave properties expressed by de Broglie wave. Usually, it can be produced by accelerating heated electrons *in vacuo.* Electron beam sources include, but are not limited to, for example, an electron tube, an electron gun and the like. An electron gun is an apparatus for emitting an electron beam in a specific direction, comprising a negative pole for emitting an electron and a positive pole for acceleration. Usually, it uses a heat negative pole and a number of circular plates or circular electrode having pore in the center on the way to the positive pole in an coaxial manner to produce a beam having a desired width and parallelism. It also uses cold negative pole release or gas discharge.

For the above-described ultraviolet irradiation, measurement is made by actinometer. Ultrasonication exposure is measured by a calorimeter or indicated by an input electric power amount or the amount of hydrogen peroxide produced. An electron beam is measured by electric current measurement or measured in the same manner as the gamma-ray. X-rays are measured in the same manner as the gamma-ray, using a measureing device such as cavity ionization chamber, calorimeter, film dosimeter, PMMA dosimeter and the like.

As used herein the term "vacuum" refers to a pressure of $1 \times 10^{-1}$Pa or less. As used herein the term "under reduced pressure" refers to a pressure of the range between $1 \times 10$Pa to $1 \times 10$Pa.

As used herein the term "atmosphere" is used in the same manner as commonly used, and usually refers to what consists of 78% nitrogen, 21% oxygen, 1%argon, 0.03% carbon dioxide, about 0.3% water vapor.

As used herein, the term "in oxygen" refers to an environment having greater oxygen concentration than the air. Preferably, "in oxygen" refers to the existence of substantially 100% oxygen.

As used herein ther term "in water" refers to a reaction in a solvent substantially consisting only of $H_2O$. Water include tap water, distilled water, ion exchanged water and the like.

As used herein the term "amphipathic molecule solution" in which a radical reaction is performed, may or may not be substantially the same amphipathic molecule solution used in the decellularization.

As used herein, the "physiologically active substance" refers to a substancewhich acts on cells or tissue. Physiologically active substances include cytokines and growth factors. Physiologically active substances may be naturally occurring or synthetic. Preferably, a physiologically active substance is one that is produced by cells or one having an action similar to that. As used herein, physiologically active substances may be in the form of a protein or nucleic acid or in other forms. When cytokines actually exert an action, the cytokines are usually in the form of proteins.

As used herein, the term "cytokine" is used in its broadest sense in the art, referring to a physiologically active substance which is produced by a cell and acts on the same or a different cell. Cytokines are generally proteins or polypeptides , which have an action of controlling an immune response, an action of regulating an endocrine system, an action of regulating a nerve system, an anti-tumor action, an anti-virus action, an action of regulating proliferation, an action of regulating differentiation, and the like. As used herein, cytokines may be in the form of proteins or nucleic acids, or in other forms. When cytokines actually exert an action, the cytokines are usually in the form of proteins.

As used herein, the term "growth factor" or "cell growth factor" are used interchangeably, and refer to a substance capable of promoting or controlling cell growth. Growth factors are also referred to as proliferation factors or development factors. Growth factors may be added to a medium in cells or tissue culture and may substitute for actions of serum macromolecule substances. It has been revealed that a number of growth factors can function as factors controlling differentiation of cells in addition to cell growth.

Cytokines typically include, interleukins, chemokines, hemopoietic factors (e.g., colony stimulating factors), tumor necrosis factors, and interferons. Growth factors typically include, a platelet-derived growth factor (PDGF), an epitdermial growth factor (EGF), a fibroblast growth factor (FGF), a hepatocyte growth factor (HGF), and a vascular endothelial growth factor (VEGF), which have growth activity.

Physiologically active substances, such as cytokines and growth factors, generally have a redundancy of functions. Therefore, even cytokines or growth factors known to have a different name and function may be used in the present invention as long as it has the same function as that of a physiologically active substance of the present invention. If cytokines or growth factors have preferable activity as set forth herein, these substances can be used in a treatment method or medicament according to a preferred embodiment of the present invention.

As used herein, the term "differentiation" refers to a developmental process of a portion of an organism, such as a cell, tissue, or an organ, in which a characteristic tissue or organ is formed. The term "differentiation" is used mainly in embryology, developmental biology, and the like. An organism is formed of various tissue and organs from the division of a fertilized ovum to form an adult organism. In an early stage of development of an organism before division or in inadequate division, individual cells or cell groups have no morphological or functional feature and it is difficult to separate the cells or cell groups. This state is called "undifferentiated" . "Differentiation" also takes place at the organ level. Cells constituting an organ develop various characteristic cells or cell groups. This is called "differentiation" in an organ during the formation of an organ. Therefore, regarding regeneration in the present invention, cell differentiation means that a cell acquires a certain morphological or functional feature which it had not been possessed before the treatment. For example, in the case of a cardiac valve, when a stem cell (e.g., an embryonic stem cell or a tissue stem cell) is provided, the stem cell is differentiated into a cell which is morphologically or functionally similar to a cell or tissue existing in the cardiac valve to some degree.

As used herein, the terms "graft" and "tissue graft" are used interchangeably, referring to homologous or heterologous tissue or cell group which is inserted into a particular site of a body and thereafter forms a portion of the body. Examples of grafts include, but are not limited to, organs or portions of organs, blood vessels, blood vessel-like tissue, skin segments, cardiac valves, pericardia, dura matter, corneas, bone segments, teeth, and the like. Therefore, grafts encompass any one of these which is inserted into a deficient portion, so as to compensate for the deficiency. Grafts include, but are not limited to, autografts, allografts, and xenografts , which depend on the type of their donor.

As used herein, the term "autograft" refers to a graft which is implanted into the same individual from which the graft is derived. As used herein, the term "autograft" may encompass a graft from a genetically identical individual (e.g. an identical twin) in a broad sense.

As used herein, the term "allograft" refers to a graft which is implanted into an individual which is the same species but is genetically different from that from which the graft is derived. Since an allograft is genetically different from an individual (recipient) to which the graft is implanted, the graft may elicit an immune reaction. Such a graft includes, but is not limited to, for example, a graft derived from a parent.

As used herein, the term "xenograft" refers to a graft which is implanted from a different species. Therefore, for example, when a human is a recipient, a porcine-derived graft is called a xenograft.

As used herein, a "recipient" (acceptor) refers to an individual which receives a graft or implanted matter and is also called "host". In contrast, an individual providing a graft or implanted matter is called "donor" (provider).

With the decellularization technique of the present invention, any graft can be used. This is because a graft (e.g., tissue, an organ, or the like) decellularized by a method of the present invention retains such a tissue damage rate that does not hinder therapy (i.e., a low tissue damage rate) and adverse effects, such as elicitation of an immune reaction, calcification, and the like, are significantly suppressed. Therefore, even in conventional situations where only an autograft is available, allografts or xenografts can be used. This is one of the significant effects of the present invention which cannot be achieved by conventional techniques.

As used herein, the term "subject" refers to an organism to which treatment of the present invention is applied and is also referred to as a "patient". A patient or subject may be preferably a human.

Cells optionally used in the method or tissue graft of the present invention may be derived from a donor genetically identical to the recipient (autologous cells); a donor genetically different from, though of the same species as the recipient (homologous cells); or a donor of a species different from the recipient (heterologous cells). Considering rejection reactions, autologous cells are preferable. When rejection reactions raise no problem, homologous cells may be used. Further, if cells capable of causing rejection reactions are treated to overcome the rejection reaction, such cells can be used. A method for avoiding rejection reactions is known in the art and is described in, for example, "Shin-Geka-Taikei, Shinzo-Ishoku Hai-Ishoku Gijyutsuteki, Rinriteki-Seibi-kara-Jissi-nimukete [Heart Transplant Lung Transplant From Technical and Ethical Development to Practice]" (3rd Version). Examples of such a method include use of an immunosuppressant or a steroid drug, and the like. Immunosuppressants for preventing rejection reactions currently include "cyclosporine" (SANDIMMUNE/NEORAL), "tacrolimus" (PROGRAF), "azathioprine" (IMURAN), "steroid hormone" (prednine, methylprednlne), and "T-cell antibodies" (OKT3, ATG, etc.). A method which is used for preventive immunosuppressive therapy in a number of facilities in the world is three-drug therapy using "cyclosporine, azathioprine, and steroid hormone". An immunosuppressant is preferably, but not necessarily, administered concomitantly with a medicament of the present invention. Therefore, an immunosuppressant may be administered before or after a regeneration/therapeutic method of the present invention as long as an immunosuppression effect can be achieved.

Cells used in the present invention may be derived from any organism (e.g., vertebrates and invertebrates). Preferably, cells derived from vertebrates are used. More preferably, cells derived from mammals (e.g., primates, rodents, etc.) are used. Even more preferably, cells derived from primates are used. Most preferably, cells derived from humans are used.

Combinations of a subject targeted by the present invention and decellularized tissue include, but not limited to, for example, implantation to heart suffering from a heart disease (e.g., ischemic heart diseases); pericardia patch, implantation of dura matter in brain surgery; implantation of a blood vessel to treat cardiac infarction, a lower limb, an upper limb, or the like; implantation of a bone to a patient suffering from bone fracture or bone failure; implantation of cornea of the present invention to a patient having an injured cornea, and the like.

Tissues targeted by the present invention may be any organ or part of an organism or may be derived from any kind of organism. Organisms targeted by the present invention include vertebrates or invertebrates. Preferably, organisms targeted by the present invention are mammals (e.g. , primates, rodents, etc.). More preferably, organisms targeted by the present invention are primates. Most preferably, organisms targeted by the present invention are humans.

Attention has been directed to blood vessel regeneration therapy using self cells, and a method for implanting tissues can be carried out and are well known in the art. In cardiovascular surgery area, various prosthetic valves or vascular prostheses or the like are used. However, a number of problems arise, such as their durability or necessity of anticoagulant therapy, and associated hemorrhagic tendency and susceptibility to infection, and the like. Therefore, there is an expectation for regenerative medical engineering. SHIN'OKA et al. prepared a regenerated blood vessels by culturing smooth muscle cells from a vein of a foot of a 4-year old female with pulmonary artery deficiency on a bioabsorbable high molecular weight polymer. Subsequently, implantation was carried for the regenerated blood vessel (Toshiharu SHIN'OKA, Yasuharu Imai, Kazuhiro Seo, et al.; "Tissue- Enjinearinnguniyoru-Shinkekkanzairyo- no- Kaihatsu, Oyo [Development and Application of Cardiovascular Material in Tissue Engineering]", Nichi Shinzo Kekkan Gekaishi, 2000; 29:38). In tissue engineering for the cardiovascular system, implanted cells or structures can make direct contact with blood in a blood vessel and are therefore supplied with oxygen and nutrients immediately after implantation. Thus, implanted cells or structures are considered to be under advantageous conditions. Particularly, the use of self cells (cell replacement) has numerous advantages, for example:

1.Removal of the possibility of a rejection reaction.
2.No necessity of considering a donor.
3. Expectation of long durability due to utilization of living tissue.
4.No residual foreign matter since a scaffold polymer is completely biodegraded when cells complete the extracellular stroma.
5.Excellent antithrombogenicity due to eventual coverage with endothelia and no necessity of anticoagulant therapy after implantation.
6.Expectation of growth due to autologous tissue.

At present, self cells are used in the right arterial system within a blood pressure range of around that of the pulmonary artery pressure. However, self cells can be appropriately applied for use with an aortic pressure, artery grafts for valve tissue or AC bypass, tendinous cord tissue, or the like ("Junkanki-Shikkan-no-Saishin-Tiryo 2002-2003 [Up-to-date Therapy for Cardiovascular diseases 2002-2003]": Nankodo, p.29, published in 2002).

General use of prosthetic valves is well known in the art. The present invention is also carried out based on this well-known subject. For example, stentless heterologous tissue valves are known. In heterologous tissue valves, the presence of a stent reduces the effective area of the valve port, leading to calcification or degeneration of valve leaflets. Recently, by utilizing the morphology of the base portion of the porcine aorta, a stentless heterologous tissue valve stent without a stent has attracted attention as a prosthetic valve for the aortic valve (Gross C., et al., Ann. Thorac. Surg., 68:919, 1999). It is considered that the absence of a stent results in a small pressure difference across the valve even when a small-sized valve is unavoidably used and is also effective for post-operative enlargement of the left ventricle. Further, the elasticity of the base portion of the aorta is maintained, stress to the cusp is small, and the durability can be expected to be improved as compared to a tissue valve with a stent. Further, stentless heterologous tissue valves can be used in the case of endocarditis, which is due to infection or prosthetic valve infection. At present, substantially satisfactory intermediate-term postoperative results of stentless heterologous tissue valves have been reported in the USA and Europe, and long-term results can be expected to be satisfactory (Gross C., et al., Ann. Thorac. Surg., 68: 919, 1999).

BEST MODE FOR CARRYING OUT THE INVENTION

(Preferable embodiments)

Hereinafter, preferred embodiments of the present invention will be described. The following embodiments are provided for a better understanding of the present invention and the scope of the present invention should not be limited to the following description. It will be clearly appreciated by those skilled in the art that variations and modifications can be made without departing from the scope of the present invention with reference to the specification.

In one aspect, the present invention provides a decellularized tissue comprising a biocompatible macromolecule. The Biocompatible macromolecule may be comprised in any state in the decellularized tissue, and preferably it is advantageous that the macromolecule to be bound via covalent linkage. Accordingly, those skilled in the art can appropriately include biocompatible macromolecules into a decellularized tissue depending on the state to be included using well known technology in the art. Such linkage may be performed by means of a crosslinking reaction. The Decellularized tissue to which biocompatible macromolecules of the present invention may be any decellularized tissue subjected to any method. The present invention has discovered that inclusion of a biocompatible macromolecule into a decellularized tissue unexpectedly and significantly enhances the tissue strength thereof. Accordingly, biocompatible macromolecule of the present invention provides a possibility of use in an application which have not been available to date due to the problem of strength. Such reinforcement of strength is preferably, for example, in terms of the state without biocompatible macromolecule, 101% or greater, more preferably 110% or greater , still more preferably, 120% or greater, yet more preferably, 150% or greater, most preferably 200% or greater.

In a preferable embodiment, the decellularized tissue of the present invention is characterized in that a) a cell redisual rate of the tissue is less than a level at which an immune reaction is elicited in an organism; and b) the tissue is not damaged to such an extent that hinders the tissue from exhibiting a function which was possessed by the tissue when the tissue was not decellularized. Cells remaining in the tissue is a cause of calcification, and in addition, in the case of transplanting a tissue from other than that of a self, there is a possiblity of raising undesired immunological reactions by the remaining cells, in case of transplanting a tissue from other than self. Therefore, in the decellularized tissue of the present invention, it is necessary to lower the level of remaining cells than that which raises immunological reactions *in vivo.* Furthermore, it is preferable to remove cells as much as possible. In the present invention, cells subjected to damage is significantly reinforced, which is one of the effects attained thereby.

In a preferable embodiment, biocompatible macromolecules used in the present invention are treated so as to coat the tissue. Such coating may be performed by means of well known technology in the art, and for example, but not limited to, a method of immersing a decellularized tissue in such a biocompatible macromolecule solution, or a method of coating by spraying.

In a more preferable embodiment, the biocompatible macromolecule of the present invention is advantageously crosslinked to the tissue. Although not wishing to be bound by any theories, crosslinkage reinforces tissue organization properties of the decellularized tissue.

Such crosslinkage is performed by for example, a radical reaction (for example, ultraviolet irradiation, exposure to a free radical source, ultrasonication, x-ray irradiation, gamma-ray irradiation and electron beam irradiation and the like).

In a preferable embodiment, biocompatible macromolecules used in the present invention may be biodegradable. Examples of such biodegradable macromolecules include, but are not limited to, for example, polyvinyl alcohol, polyglycolic acid, polylactic acid, and the like.

In a preferable embodiment, biocompatible macromolecules used in the present invention may comprise a macromolecule selected from the group consisting of polyvinyl alcohol, polyvinyl pyrrolidone, elastin and the mixture of the two or more thereof. More preferably, biocompatible macromolecules used in the present invention may comprise polyvinyl alcohol or polyvinyl pyrrolidone.

In a preferable embodiment, polyvinyl alcohol used in the present invention may be in the range of molecular weight of 500-200,000. More preferably, polyvinyl alcohol used is of molecular weight of 500 to 100,000, and more preferably, of 500-10,000.

In a preferable embodiment, polyvinyl pyrrolidone used in the present invention may be in the molecular weight range of 500-200,000. More preferably, polyvinyl alcohol used is of molecular weight of 1,000 to 100,000, and more preferably, of 10,00-50,000 (for example , 40,000).

In a preferable embodiment, a biocompatible macromolecule used in the present invention is used at the concentration of 1 (w/v) to 50% (w/v). More preferably, the biocompatible macromolecule may preferably be used at from 5%(w/v) to 30% (w/v). Still more preferably, the biocompatible macromolecule may preferably be used at from 10% (w/v) to 20% (w/v).

The decellularized tissue of the present invention is used in transplantation therapy, and thus it is preferable that the tissue or organ is not damaged such that the function possessed thereby prior to the treatment (decellularization) is maintained. Such effects are not substantially damaged by the radical reaction according to the present invention. The presence or absence of such hinderance can be determined by confirming that the extracellular matrix of the tissue is not substantially degenerated. Therefore, the tissue of the present invention may be characterized in that the extracellular matrix substantially remains. The presence of extracellular matrices can be confirmed by staining using a marker specific thereto. As described above, the tissue of the present invention has excellent physical strength, pressure-resistant property, and the like and therefore is preferably used as a scaffold for re-cellularization (or cell replacement) in a recipient. Conventionally, there is a method for reducing a cell redisual rate in tissue (Koide A., Hayashi T., eds (2000); "Basic and Clinical Studies of Extracellular Matrix (ECM); Aichi Shuppan Co., Ltd., Tokyo, Japan CAN: 133. 333569). However, when a conventional method is used to reduce a cell redisual rate to 30% or less, tissues are damaged to such an extent that the tissue cannot withstand organ implantation. The present invention achieves a significant effect in that cell redisual rate can be reduced while tissue or an organ is not damaged to such an extent that hinders the tissue or organ from exhibiting a normal function which was possessed by the tissue or organ before treatment (decellularization). The present invention can be used with cells or without a cell.

In order to present the above-described adverse effects, the cell redisual rate of the decellularized tissue according to one embodiment of the present invention is typically about 30% or less, more preferably about 25% or less, still more preferably about 20% or less, still even more preferably about 15% or less, still even more preferably about 10% or less, and most preferably about 5% or less. The decellularized tissue of the present invention is characterized in that although the cell redisual rate thereof is low, the tissue damage rate thereof is as low as a level that permits use in clinical applications. Unless a function originally possessed by the tissue (before treatment) can be exhibited, the tissue cannot be used in clinical applications no matter how low the cell redisual rate is. Therefore, as the tissue damage rate of the tissue is decreased, while keeping the above-described cell redisual rate, the tissue is more preferable. In other words, the tissue has to maintain a low tissue damage rate which permits clinical applications.

The smaller the tissue damage rate that permits the tissue to be used in clinical applications, the more preferable the tissue of the present invention is. The tissue damage rate may be typically about 50% or less, representatively about 40% or less, preferably about 30% or less, more preferably about 25% or less, still more preferably about 20% or less, still even more preferably about 15% or less, still even more preferably about 10% or less, and most preferably about 5% or less. Even though the tissue damage rate is decreased in this manner, it is not possible to use the tissue or an organ which is damaged to such an extent that the tissue or organ cannot exhibit an originally possessed function thereof. Therefore, in order to achieve the objective of the present invention, it is preferable that the tissue of the present invention is not damaged to such an extent that the tissue is hindered from exhibiting an originally possessed function thereof. It is possible to evaluate the tissue damage rate to determine whether or not the tissue can exhibit an originally possessed function thereof. The tissue damage rate can be evaluated by the above-described method.

Alternatively, the decellularized tissue of the present invention can be determined by evaluating the survival rate of extracellular matrices. The survival rate of extracellular matrices may be, for example, about 50% or more, preferably about 70% or more, more preferably about 80% or more, and even more preferably about 90% or more.

In one embodiment, the decellularized tissue of the present invention has such a tissue strength that permits clinical applications. A sufficiently high level of tissue strength is an important characteristic, particularly when membrane-like tissue is used within clinical applications. Tissue strength can be generally determined by measuring tensile strength (e.g., breaking strength, stiffness, Young's modulus, etc.). In a certain embodiment, the decellularized tissue of the present invention may have a tissue strength which is at least about 75% or more of the strength which was possessed by the tissue before treatment, preferably about 80% or more, more preferably about 85% or more, still more preferably about 90% or more, or alternatively may have a tissue strength greater than or equal to that which was possessed by the untreated tissue (originally possessed tissue strength).

In one embodiment, the decellularized tissue of the present invention has a tissue strength that permits clinical applications. A sufficiently high level of tissue strength is an important characteristic, particularly when membrane-like tissue is used in clinical applications. Tissue strength can be generally determined by measuring tensile strength (e.g., breaking strength, stiffness, Young's modulus, etc.). In a certain embodiment, the decellularized tissue of the present invention may have a tissue strength which is at least about 75% or more of the strength which was possessed by the tissue before treatment, preferably about 80% or more, more preferably about 85% or more, still more preferably about 90% or more, or alternatively may have a tissue strength greater than or equal to that which was possessed by the untreated tissue (for example, greater than originally possessed tissue strength such as 110% or more, 120% or more, 150% or more, 200% or more of that originally possessed).

As used herein, the term "tissue strength of untreated tissue" refers to tissue strength which was possessed by tissue before (i.e. inanative state) treatment (for example, decellularization treatment of the present invention and treatment by means of biocompatible molecule of the present invention). Sufficiently strong tissue strength is a preferable property for applying it to a tissue such as valvular tissue, tubular tissue and the like.

In the case of luminal tissue, tissue strength can be represented by a β value. A method for calculating a β value is heretofore described in detail and is also illustrated in examples below. In a certain embodiment, the decellularized tissue of the present invention has a tissue strength having a β value of about 15 or more, preferably a β value of about 18 or more, more preferably a β value of about 20 or more, and still more preferably a β value of about 22 or more. In another embodiment, the decellularized tissue of the present invention may have a β value which is at least about 75% or more of that which was possessed by the tissue before treatment, preferably about 80% or more, more preferably about 85% or more, still more preferably about 90% or more, or alternatively may have a β value greater than or equal to that which was possessed by the untreated tissue (originally possessed β value). As used herein, the term "characteristic of untreated tissue" refers to a characteristic which was possessed by tissue before treatment (e.g., treatment with a 1,2-epoxide polymer of the present invention). For example, untreated tissue is naturally occurring.

The decellularized tissue of the present invention may be tissue which is derived from any part of the body if the tissue is intended to be used in clinical applications. In a certain embodiment, the decellularized tissue of the present invention may require a physical structure. In order to maintain physical structure, only a structure such as cytoskeleton and the like are required, while intracellular components, such as plasma components and the like are not necessarily required. Also, optionally required cells may be additionally provided to decellularized tissues or may be internally supplied from a host to which the tissue is implanted. In a certain embodiment, the decellularized tissue of the present invention may be derived from an organ selected from blood vessels, blood vessel-like tissue, cardiac valves, pericardia, dur a matter, corneas, and bones.

In another embodiment, the decellularized tissue of the present invention may be derived from an organ selected from cardiovascular tissue (e.g., blood vessels, blood vessel-like tissue, cardiac valves, and pericardia).

The decellularized tissue of the present invention may be tissue derived from any organism if the tissue is suitable for an intended clinical application. Therefore, the tissue of the present invention may be derived from any organism (e.g., vertebrates and invertebrates). When intended to be applied to a human, vertebrate-derived tissue is preferably used, and more preferably a tissue derived from a mammal (e.g., a primate, a rodent, or the like) is used. When intended to be applied to a human, primate-derived tissue is more preferably used. When intended to be applied to a human, porcine-derived tissue is more preferably used. This is because porcine-derived tissue has a size similar to that of a human. When intended to be applied to a human, human-derived tissue is most preferably used. The decellularized tissue or tissue graft of the present invention preferably has a size similar to that of a human and a physical characteristic similar to that of a human, when the tissue or tissue graft is derived from organisms other than a human (e.g., swine).

In the present invention, the decellularized tissue of the present invention may be applied to any part of the body of an organism. Therefore, the decellularized tissue may be applied to a part of the body from which the decellularized tissue was derived or other parts of the body. As shown in the Examples, it was demonstrated that the desired effect (e.g., regeneration, self organization) of the present invention can be achieved for any part of the body to which the decellularized tissue of the present invention is applied, no matter whether or not the part is a site from which the tissue was derived. Therefore, the present invention has a considerable advantageous utility, in that the present invention can in principle be applied to any implantation operation and/or regeneration operation. Examples of parts to which the decellularized tissue of the present invention is applied, include, but are not limited to, skin, blood vessel, cornea, kidney, heart, liver, umbilical cord, intestine, nerve, lung, placenta, pancreas, brain, peripheral limb, retina, valve, epithelial tissue, connective tissue, muscular tissue, nervous tissue, and the like.

In another aspect of the present invention, a tissue graft containing the decellularized tissue of the present invention is provided. In this tissue graft, recipient-derived cells are disseminated and cultured in the above-described decellularized tissue so that desired tissue structure is formed. The tissue graft of the present invention may be intended to be implanted into any tissue in the body as long as the tissue graft is of tissue intended for a clinical application. In a certain embodiment, the tissue graft of the present invention may require a physical structure. In order to maintain physical structure, recipient-derived cells may be provided to the above-described decellularized tissue before implantation. The recipient-derived cells may be internally supplied from a host to which the tissue graft is implanted. In a certain embodiment, the tissue graft of the present invention may be derived from an organ selected from blood vessels, blood vessel-like tissue, cardiac valves, pericardia, duramatter, corneas, and bones. In another embodiment, the tissue graft of the present invention may be derived from an organ selected from cardiovascular tissue (e.g., blood vessels, blood vessel-like tissue, cardiac valves, and pericardia).

The tissue graft of the present invention may contain tissue derived from any organism if the tissue is suitable for an intended clinical application. Therefore, the tissue graft of the present invention may contain tissue derived from any organism (e.g., vertebrates and invertebrates). When intended to be applied to a human, vertebrate-derived tissues are preferably used for the tissue graft of the present invention, and more preferably tissue derived from a mammal (e.g., a primate, a rodent , or the like) is used for the tissue graft of the present invention. When intended to be applied to a human, primate-derived tissue is more preferably used for the tissue graft of the present invention. When intended to be applied to a human, porcine-derived tissues are more preferably used for the tissue graft of the present invention. This is because porcine-derived tissue has a size similar to that of a human. When intended to be applied to a human, human-derived tissue is most preferably used.

Recipient-derived cells used in the tissue graft of the present invention may be any cells that are suitable for clinical applications. Therefore, the cell may be selected from the group consisting of vascular endothelial cells, smooth muscle cells, fibroblasts, blood cells, and precursor cells and somatic stem cells capable of differentiating into those cells. Preferably, the cell may be a cell capable of exhibiting a desired function at a site at which the cell is implanted.

In the present invention, the tissue graft of the present invention may be applied to any part of the body of an organism. Therefore, the tissue graft may be applied to a part of the body from which the tissue graft was derived or other parts of the body. As shown in the Examples, it was demonstrated that the desired effect (e. g. , regeneration, self organization) of the present invention can be achieved fox any part of the body to which the tissue graft of the present invention is applied, no matter whether or not the part is a site from which the tissue was derived. Therefore, the present invention has a considerable advantageous utility, such that the present invention can in principle be applied to any implantation operation and regeneration operation. Examples of parts to which the tissue graft of the present invention is applied, include, but are not limited to, skin, blood vessel, cornea, kidney, heart, liver, umbilical cord, intestine, nerve, lung, placenta, pancreas, brain, peripheral limb, retina, valve, epithelial tissue, connective tissue, muscular tissue, nervous tissue, and the like.

In another aspect of the present invention a membrane-like tissue graft is provided. This membrane-like tissue graft comprises a) the decellularized tissue of the present invention. Here, the recipient-derived cells are disseminated and cultured in the decellularized tissue so that desired tissue structure is formed. Cells are not necessarily included therein. Preferably, it is advantageous not to have a cell, because no immunological rejection reaction will occur.

In another aspect, the present invention provides a method for producing a tissue graft, comprising the steps of: a)providing decellularized tissue comprising a biocompatible macromolecule into an organism; b)allowing a self cell in the organism to infiltrate the decellularized tissue; and c) incubating the tissue within the organism for a time sufficient for the cell to differentiate. Any tissue may be used in the present method. The decellualization step may be any method in the art, and includes but is not limited to, treatment with surfactant or detergent (SDS, Triton X-100, sodium cholate, sodium deoxycholate, beta-octyl-D-glucoside and the like), an amphipathic molecule (for example, PEG, glycerin and the like) and the like; ultrasonication treatment; change in static pressure or atmospheric pressure; change in osmotic pressure of the solution, and the like.

Preferably, the decellularization step used in the present invention may include, immersion in a solution comprising an amphipathic molecule in a non-micelle state, or a surfactant solution. More preferaly, it is desirable that treatment in a amphipatic molecule in a non-micelle state is desirable. Treatment using an amphipatic molecule in a non-micelle state does not reduce the strength of the decellularized tissue.

In a preferable embodiment, the step of exposing the biocompatible macromolecule used in the present invention includes subjecting the biocompatible macromolecule to a radical reaction (for example, ultraviolet irradiation, exposure to a free radical source, ultrasonication, x-ray irradiation, gamma-ray irradiation and electron beam irradiation and the like). Preferably, gamma-ray irradiation is used.

In one preferable embodiment, exposure dose of gamma-ray irradiation used in the present invention is within the range of 10-300kGy. More preferably, gamma-ray irradiation dose is within the range of 50-100kGy. Although not wishing to be bound by any theories, such an exposure dose is appropriate for enhancing tissue strength. It is appropriate to set the upper limit for the dose capable of maintaining the tissue strength depending on the tissue that is issued. In view of the tissue strength, there are some cases where 60kGy has obtained greater strength than 100kGy, however, the present invention is not limited thereto.

As used herein, gamma irradiation may be conducted under a circumstance selected from the group consisting of in vacuum, in oxygen, in nitrogen, in the atmosphere, in water , in an amphipathic molecule solution and a combination thereof. Preferably, the irradiation is conducted in the atmosphere.

In a preferable embodiment, gamma-ray irradiation used in the present invention may be conducted in the range of for 0.5 to 240hours, preferably between 1 to 24 hours, still more preferably, 3-8 hours. Although not wishing to be bound to any theories, if unit exposure dose per unit time is increased by using stronger gamma-ray source, then it can shorten the time required for irradiation. However, if the gamma-ray irradiation that is used is too strong, then it may be possible for adverse effects to occur on the tissue. Moreover, in order to extend the time required, a weaker gamma-ray source may be used. Although not wishing to be bound to any theories, if a irradiation source that is to weak is used, it requires too long to cause irradiation, and in addition, it may be possible that the tissue per se may receive damages due to other reasons.

The present invention may attain effects of decellularization reinforcement of biocompatible macromolecule by means of any decellularization technology. Such a decellularization technology includes those using surfactants such as SDS, Tris, and the like, or those having an amphipatic molecule, e.g., 1,2-epoxide polymer such as PEG. Preferably, decellularuzation treatment using PEG is preferable but SDS may also be used. In one preferred embodiment, the average molecular weight of PEG is between 200 and 6000, and preferably between 200 and 2000. PEG used in the present invention may have various average molecular weights. By changing treatment time (immersion time) depending on the average molecular weight, a desired effect (e. g. , decellularization) can be achieved. In one preferred embodiment, the average molecular weight of PEG used in the present invention is between 1000 and 2000. In another preferred embodiment, the average molecular weight of PEG used in the present invention is between 1500 and 2000. In another embodiment, the average molecular weight of PEG used in the present invention is smaller than or equal to 1000. Alternatively, in one preferable embodiment, PEG having an average molecular weight of 1,000 is more advantageous.

Although not wishing to be bound by any theory, in general, as the average molecular weight of PEG used is increased, the treatment time has to be decreased, although the present invention is not limited thereto. This is because as the average molecular weight of PEG used is increased, the effect of extracting intracellular components increases. Therefore, in one embodiment, the tissue may be immersed in a 1,2-epoxide polymer-containing solution for 30min to several weeks (for example, ten days), more generally, 30min to several days. A minimum time required in the immersing step varies depending on the amphipathic molecule used (e.g., a 1,2-epoxide polymer, such as PEG), but can be determined by those skilled in the art without undue experimentation. Therefore, the step of immersing the tissue in a solution containing an amphipathic molecule, such as a 1,2-epoxide polymer, may be performed for less than 30min. Alternatively, the immersion step using a solution containing an amphipathic molecule, such as a 1,2-epoxide polymer, may be performed for more than several hours (alternatively, sixty days). A minimum time can be obtained as follows: various treatment times are provided in control experiments; at each time point, the state of treated tissue is determined by measuring the tissue damage rate, the cell redisual rate, or the like of the tissue are measured by methods as described herein; and based on the result, it is determined whether or not the treated tissue has an appropriate characteristic. In the method of the present invention, the step of immersing tissue in the solution may be performed under any condition and may be performed typically at a temperature of between 0°C and 42°C, at room temperature (between about 15°C and about 25°C), or at 37°C. This step may be performed at a temperature of more than 37°C.

In the method of the present invention, the 1,2-epoxide polymer (e.g., PEG) may be present in the solution at any concentration and may be preferably present at a concentration of 1g/ml or more. Such a substance is expected to act as a radical scavenger for radical reactions (in particular, physical radical source such as gamma-rays). Alternatively, when considering efficiency of radical reactions, the concentration of PEG solution may be any concentration, but is preferably between about 60 % and about 100%, and for example, those having about 80% may be used but are not limited thereto.

Note that when PEG (molecular weight: 1000) is used as a material reagent, it is solid at room temperature and therefore may be dissolved in water. In the method of the present invention, the 1,2-epoxide polymer (e.g., PEG) may be dissolved in any solvent and may be preferably dissolved in an aqueous medium, more preferably in physiological saline, PBS (phosphate buffered saline), or other solutions containing salts. Such a solution may be any solution in which an amphipathic molecule used in the present invention is not micellar at a concentration which is usually used for the solution. The solution having a non-micellar amphipathic molecule (e.g., a 1,2-epoxide polymer) is pr-eferably sterilized. A non-micellar amphipathic molecule (e.g., a 1,2-epoxide polymer) used in the present invention is preferably biocompatible. Such a biocompatible, non-micellar amphipathic molecule (e.g., a 1,2-epoxide polymer) includes, but is not limited to, polymers which are biocompatible and are of the medicament grade, for example, PEG, segmented polyurethane, silicone, MMA (α-methylmethacrylate (contact lens material), etc.), and PTFE (polytetrafluoroethylene) (e.g., Teflon (registered trademark), Dacron, etc.). Such a biocompatible 1,2-epoxide polymer is described in, for example, Japanese Pharmacopoeia (or counterparts in other countries) or may be a polymer approved by the Health, Labor and Welfare Ministry (or counterparts in other countries).

Preferably, the method of the present invention further comprises washing the tissue immersed in the solution. The washing step can be performed using any liquid which is physiologically suitable (e.g., physiological saline, PBS (phosphate buffered saline), etc.). In a preferred embodiment, the washing step in the present invention is performed with PBS. The wash solution is preferably sterilized. More preferably, washing solution includes antibiotics (for example, penicillin, cephalosporin, streptomycin, tetracyclin, erythromycin, vancomycin, ciprofloxacin, rinezuride and the like). The washing step may be performed under any condition and may be performed typically at a temperature of between 0°C and 42°C, at room temperature (about 15°C and about 25°C), or at 37°C. The washing step may be performed at a temperature of more than 37°C. In the method of the present invention, the washing step may be performed for any period of time as long as the solution containing an amphipathic molecule, such as a 1,2-epoxide polymer, can be sufficiently removed and may be usually performed for 0.5days to 5days. Preferably, in the washing step, a wash solution (e.g., PBS) may be changed several times.

Tissues used in the method of the present invention may be tissue derived from any organism if the tissue is suitable for an intended clinical application. Therefore, tissue used in the method of the present invention may be derived from any organism (e.g., vertebrates and invertebrates). When intended to be applied to a human, vertebrate-derived tissues are preferably used within the method of the present invention. More preferably, tissue derived from a mammal (e.g., a primate, a rodent, or the like) is used as tissue within the method of the present invention. When intended to be applied to a human, primate-derived tissue is more preferably used as tissue used in the method of the present invention. When intended to be applied to a human, porcine-derived tissue is more preferably used. This is because porcine-derived tissue has a size similar to that of a human. When intended to be applied to a human, human-derived tissue is most preferably used.

Tissue used in the method of the present invention may be any tissue in the body as long as the tissue is intended to be used in clinical applications. In a certain embodiment, the tissue used in the method of the present invention may require a physical structure or physical properties. In order to maintain physical structure or physical properties, only structure such as extracellular matrices and the like are required, while intracellular components such as plasma components, cell membrane components, and the like, are not necessarily required. Also, optionally required cells may be additionally provided to the decellularized tissue or may be internally supplied from a host to which the tissue is implanted. In a certain embodiment, tissue used in the method of the present invention may be derived from an organ selected from blood vessels, blood vessel-like tissue, cardiac valves, pericardia, dura matter, corneas, and bones.

In another embodiment, the decellularized tissue of the present invention may be derived from an organ selected from cardiovascular tissue (e.g., blood vessels, blood vessel-like tissue, cardiac valves, and pericardia).

In a preferred embodiment, the method of the present inventionmay further comprise performing chemical treatment. Here, the chemical treatment may be one other than the step of immersing tissue in a solution containing a non-micellar amphipathic molecule (e.g., a 1,2-epoxide polymer, such as polyethylene glycol) in the method of the present invention. Alternatively, the chemical treatment may be the same as the step of immersing tissue in a solution containing a non-micellar amphipathic molecule (e.g., a 1,2-epoxide polymer, such as polyethylene glycol) in the method of the present invention (i.e., repeating the immersing step). Therefore, for example, in the methods of the present invention when the chemical treatment is performed in addition to the step of immersing the tissue in a solution containing polyethylene glycol having an average molecular weight of 1000 to 2000, the chemical treatment comprises the step of immersing the tissue in a solution other than the solution containing polyethylene glycol having an average molecular weight of 1000 to 2000 (including, but not limited to, for example, a solution containing DNase (e.g., DNaseI), a glutaraldehyde-containing solution, a solution containing polyethylene glycol having another average molecular weight, a solution containing another non-micellar amphipathic molecule (e.g., a 1,2-epoxide polymer), and the like)). Alternatively, for example, when the chemical treatment is performed in addition to the step of immersing the tissue in the solution containing polyethylene glycol having an average molecular weight of 1000 to 2000, the chemical treatment may comprise repeating the step of immersing the tissue in the solution containing polyethylene glycol having an average molecular weight of 1000 to 2000.

In one embodiment, the above-described chemical treatment may be a treatment with a di-functional molecular cross-linking agent (e.g., glutaraldehyde or a derivative thereof). The treatment with a di-functional molecular cross-linking agent is intended to chemically cross-link a protein component contained in ECM or tissue cells to increase physical strength. Therefore, if this purpose can be achieved, any di-functional molecular cross-linking agent can be used. Among such di-functional molecular cross-linking agents, some agents which can be actually used for fixation of tissues (valve graft) include, but are not limited to, for example, cyanimide, 1-ethyl-3-(3-dimethylaminopropyl)carbodiimidehydrochlor ide (EDC), epoxy(poly(glycidylether)) or photocross-linking agent PhotoMix™ (Sulzer Carbomedics Co., Ltd.) (see, Biomaterials (2000) 21:2215-2231).

In another embodiment, the chemical treatment may comprise treatment with DNase, such as DNaseI. DNA components undesirable for implantation can be removed by means of the treatment with DNase. Therefore, the treatment with DNase is preferable . Such DNase may be any DNase and may be preferably DNaseI. With the DNase treatment, DNA, which is a charged polymer substance, can be removed. DNA has the possibility of eliciting an immune reaction. Therefore, by removing DNA, an advantage can be provided.

Treatment with a nuclease (e.g., DNase) may be conducted under any conditions, and may combine a number of conditions such as a pressurized condition, agitation conditions and the like. Concentrations used are for example, 1U/ml-100U/ml, and the like, and examples include, 50-75 U/ml but are not limited thereto. Nuclease treatment may be conducted for about, 0.5 to five days, and amongst them, the first several hours (for example, 1-24 hours), a pressurization condition (for example, 100-1000kPa, for example, 500 kPa) may be employed. Thereafter, agitation (for example, 100-500 RPM, preferably, 100-150RPM) may also be conducted. Before and after the nuclease treatment, washing solution as described above may preferably be used for preservaton.

In a preferable embodiment, the method for producing a decellularized tissue of the present invention includes further step of seeding cells, but is not limited thereto. Cells to be seeded may be appropriately selected by those skilled in the art depending on the conditions as described hereinabove.

The present invention also provides decellularized tissues obtained by the decellularization method of the present invention. This decellularized tissue may preferably have the above-described cell redisual rate and/or tissue damage rate and/or tissue strength. Before the decellularization method of the present invention is provided, it was not possible to provide decellularized tissue having the above-described cell redisual rate and/or tissue damage rate and/or tissue strength. Thus, the decellularization method of the present invention has an unexpected advantage of providing decellularized tissue having a characteristic which could not be provided by conventional methods.

In another aspect of the present invention provides a method of regenerating tissues. This method comprises the steps of a) providing the decellularized tissue comprising a biocompatible macromolecule of the present invention into an organism; and b) incubating the tissue for a sufficient time for regeneration in the organism. Optionally, the present inventive method may further comprises the step of providing a physiologically active substance inducing differentiation of the cell and/or providing the decellularized tissue with a cell or it is not necessary to provide the decellularized tissue with a cell. Preferably, physiologically active substances necessary for maintenance or differentiation of a desired tissue are used. Physiologically active substance may be derived from the body or externally derived. In the case of those derived from the body, it is understood that no substantial operation is necessary for exposure of an appropriate physiologically active substance by lapsing a certain period of time after transplantation. Physiologically active substances include, but are not limited to, for example, HGF, VEGF, FGF, IGF, PDGF and EGF.

Preferably, the cell may be a blood vessel cell or a blood vessel-like cell. More preferably, the cell may be derived from the recipient. Preferably, the tissue may be selected from the group consisting of blood vessels, blood vessel-like tissue, cardiac valves, pericardia, duramatter, corneas, and bones. In a preferred embodiment, the tissue and the cell may be derived from the same host. In another embodiment, the tissue and the cell may be derived from homologous hosts. In another embodiment, the tissue and the cell may be derived from heterologous hosts. When the recipient and the cell are homologous or heterologous, a rejection reaction is expected. Therefore, the recipient and the cell are preferably derived from the same host. When a rejection reaction does not raise a problem, the recipient and the cell may be homologous or heterologous. If the cell which elicits a rejection reaction is optionally treated to overcome the rejection reaction, the cell can be used. A method for overcoming a rejection reaction is known in the art and is described in, for example, "Shin-Geka-Taikei, Shinzo-Ishoku · Hai-Ishoku Gijyutsuteki, Rinriteki-Seibi-kara-Jissi-nimukete [Heart Transplant Lung Transplant - From Technical and Ethical Development to Practice]" (3rd Version). Such a method includes, for example, use of an immunosuppressant or a steroid, and the like. Immunosuppressants for preventing rejection reactions currently include "cyclosporine" (SANDIMMUNE/NEORAL), "tacrolimus" (PROGRAF), "azathioprine" (IMURAN), "steroid hormone" (prednine, methylprednine), "T-cell antibodies" (OKT3, ATG, etc.). A method which is used for preventive immunosuppressive therapy in a number of facilities in the world is three-drwg therapy using "cyclosporine, azathioprine, and steroid hormone". An immunosuppressant is preferably, but not necessarily, administered concomitantly with a medicament of the present invention. Therefore, an immunosuppressant may be administered before or after a regeneration method of the present invention as long as an immunosuppression effect can be achieved.

In another aspect of the present invention a method of producing a tissue graft is provided. This method comprises the steps of a) providing the decellularized tissue comprising a biocompatible macromolecule of the present invention into an organism; b) allowing self cells in the organism to infiltrate the decellularized tissue; and C) incubating the tissue for a sufficient time to differentiate the self cells. In this case, the decellularized tissue of the present invention may or may not have additional cells. The additional cells may be autologous, homologous, or heterologous. Preferably, the cell may be a blood vessel cell or a blood vessel-like cell. More preferably, the cell may be derived from a recipient. Preferably, the tissue may be selected from the group consisting of blood vessels, blood vessel-like tissue, cardiacvalves, pericardia, duramatter, corneas, and bones. In a preferred embodiment, the tissue and the cell may be derived from the same host. In another embodiment, the tissue and the cell may be derived from homologous hosts. In another embodiment, the tissue and the cell may be derived from heterologous hosts. When the recipient and the cell are homologous or heterologous, a rejection reaction is expected. Therefore, the recipient and the cell are preferably derived from the same host. When a rejection reaction does not raise a problem, the recipient and the cell may be homologous or heterologous. If the cell which elicits a rejection reaction is optionally treated to overcome the rejection reaction, the cell can be used. A method for overcoming a rejection reaction is herein described,

In a preferred embodiment, the method of the tissue graft of the present invention may further comprise d) providing a physiologically active substance capable of inducing differentiation of the above-described cell. Preferably, the physiologically active substance may be a cytokine having hematopoietic activity. In order to produce a tissue graft, the cytokine may be HGF, VEGF, FGF, or the like. The cell may be either autologous or heterologous. When the physiologically active substance is derived from self, it should be noted that lapsing a certain period of time after transplantation simply achieves the purpose of the present invention.

In another aspect, the present invention provides a tissue graft produced by the method of the present invention. Such a tissue graft has a characteristic which is not conventionally obtained with respect to the tissue strength and the decellularization rate.

In another aspect of the present invention a method for treating a subject requiring implantation of tissue or an organ or treating a subject at a risk of implantation of tissue or an organ for prophylaxis is provided. This method comprises a) providing the decellularized tissue or tissue graft comprising a biocompatible macromolecule of the present invention; and b) implanting the decellularized tissue to the subject. The decellularized tissue optionally contains additional cells. The cell may be autologous or homologous. Preferably, the tissue may be selected from the group consisting of bloodvessels, bloodvessel-like tissues, cardiac valves, pericardia, dura matter, corneas, and bones. In a preferred embodiment, the tissue may be derived from the subject. In another embodiment, the tissue maybe derived from a host homologous to the subject. In another embodiment, the tissue may be derived from a host heterologous to the subject. The therapeutic/ prophylactic method of the present invention employs tissues which are sufficiently decellularized that a tissue damage rate is reduced to such a level that permits implantation. Therefore, the tissue does not elicit a rejection reaction. In the case that a rejection reaction is elicited by the tissue or non-recipient-derived cells, treatment for overcoming a rejection reaction can be optionally performed. A method for overcoming a rejection reaction is herein described in detail. In one embodiment, tissues used in the treatment or prophylactic method of the present invention may be derived from the subject. In another embodiment, a tissue used in the treatment or prophylactic method of the present invention may be derived from any organism (e.g., vertebrates and invertebrates). Preferably, when a human is treated for therapy or prophylaxis, a vertebrate-derived tissue may be used. More preferably, when a human is treated for therapy or prophylaxis, atissuederivedfromamammal (e.g., aprimate, a rodent, etc.) may be used. Still more preferably, when a human is treated for therapy or prophylaxis , primate-derived tissue may be used. In another preferred embodiment, when a human is treated for therapy or prophylaxis, porcine-derived tissue may be used. This is because porcine-derived tissue has a size similar to that of a human. Most preferably, when a human is treated for therapy or prophylaxis, human-derived tissue may be used.

In another aspect, the present invention provides a medicament for organ implantation. This medicament comprises the decellularized tissue comprising a biocompatible macromolecule of the present invention or the tissue graft of the present invention. In a certain embodiment, the medicament of the present invention comprises tissue derived from an organ selected from blood vessels, blood vessel-like tissue, cardiac valves, pericardia, dura matter, corneas, and bones. In another embodiment, the medicament of the present invention may comprise cardiovascular tissue, for example, tissue derived from an organ selected from blood vessels, blood vessel-like tissue, cardiac valves, and pericardia.

The medicament of the present invention may comprise tissue derived from any organism as long as the tissue is suitable for an intended clinical application. Preferably, the medicament may comprise a material approved by an authority in a country in which the medicament is used. Therefore, the medicament of the present invention may comprise tissue derived from any organism (e.g., vertebrates and invertebrates). In the medicament of the present invention, when intended to apply the medicament to a human, preferably a vertebrate-derived tissue is used. More preferably a tissue from a mammal (e.g., a primate, a rodent, etc.) is used. In the medicament of the present invention, when it is intended to apply the medicament to a human, still more preferably a primate-derived tissue is used. When the medicament of the present invention is intended to be applied to a human, still even more preferably a porcine-derived tissue is used. This is because porcine-derived tissue has a size similar to that of a human. When the medicament of the present invention is intended to be applied to a human, most preferably a human-derived tissue is used. Note that when human-derived tissue is used, ethical regulations or problems have to be solved.

The medicament, tissue graft and decellularized tissue of the present invention may further comprise biocompatible material. For example, the biocompatible material may comprise at least one selected from the group consisting of silicone, collagen, gelatin, glycolic acid/lactic acid copolymers, ethylene/vinyl acetate copolymers, polyurethane, polyethylene, polytetrafluoroethylene, polypropylene, polyacrylate, and polymethacrylate. Silicone is preferable because of ease to mold. Examples of biodegradable polymers include collagen, gelatin, polymers or copolymers synthesized by non-catalytic dehydrocondensation of at least one selected from $\alpha$-hydroxycarboxylic acids (e.g., glycolic acid, lactic acid, hydroxybutyric acid, etc.), hydroxydicarboxylic acids (e.g., malic acid, etc.), and hydroxytricarboxylic acids (e.g., citric acid, etc.) or a mixture thereof, poly-$\alpha$-cyanoacrylic ester, polyamino acids (e.g., poly-$\gamma$-benzyl-L-glutamic acid, etc.), and polyanhydrides such as maleic anhydride copolymers (e.g., styrene-maleic acid copolymers, etc.), and the like. These copolymers may be any of random, block, and graft copolymers. When $\alpha$-hydroxycarboxylic acids, hydrox-ydicarboxylic acids, and hydroxytricarboxylic acids have an optical activity center within the molecule, the molecule can be in any of the D-form, L-form, and DL-form. In a certain embodiment, a glycolic acid/lactic acid copolymer may be used.

The pharmaceutical, medicament, tissue graft, and decellularized tissue of the present invention may further comprise another pharmaceutical agent. Such a pharmaceutical agent may include any pharmaceutical agent known in the field of pharmaceuticals. The medicament, tissue graft, and decellularized tissue of the present invention may comprise two or more other pharmaceutical agents. Such pharmaceutical agents include, for example, those which are described in the up-to-date version of Japanese Pharmacopoeia, US Pharmacopoeia, pharmacopoeias in other countries, and the like. The pharmaceutical agent may preferably have an effect on an organ of an organism. The pharmaceutical agent includes, for example, thrombolytic agents, vasodepressors, and tissue activating agents. The amounts of a physiologically active substance and other pharmaceutical agents and cells contained in the medicament, tissue graft, and decellularized tissue of the present invention can be easily determined by those skilled in the art, considering purposes of use, target diseases (type, severity, etc.), patient's age, weight, sex, case history, and the like.

In another aspect, the present invention relates to use of the decellularized tissue of the present invention or the tissue graft of the present invention for organ implantation and medicament production. In a certain embodiment, regarding the use of the present invention, tissue derived from an organ selected from blood vessels, blood vessel-like tissue, cardiac valves, pericardia, dura matter, corneas, and bones, may be used. In another embodiment, regarding the use of the present invention, tissue derived from an organ selected from cardiovascular tissue, such as blood vessels, blood vessel-like tissue, cardiac valves, and pericardia, may be used.

Regarding the use of the present invention, tissue derived from any organism may be used as long as the tissue is suitable for an intended clinical application. Preferably, a material approved by an authority in a country in which the medicament is used, may be used. Therefore, regarding the use of the present invention, tissue derived from any organism (e.g. , vertebrates and invertebrates) may be used. Regarding the use of the present invention, when intended to apply the present invention to a human, preferably, vertebrate-derived tissue is used. More preferably, tissue from a mammal (e.g., a primate, a rodent, etc.) is used. Regarding the use of the present invention, when intended to apply the present invention to a human, still more preferably, primate-derived tissue is used. When the present invention is intended to be applied to a human, still even more preferably, porcine-derived tissue is used. This is because porcine-derived tissue has a size similar to that of a human. When the present invention is intended to be applied to a human, most preferably, human-derived tissue is used. Note that whenhuman-derived tissue is used, ethical regulations or problems have to be solved.

The use of the decellularized tissue, graft, pharmaceutical, and medicament of the present invention is usually performed under supervision of a doctor, or without supervision of a doctor if approved by an authority and a law of a country in which the present invention is used.

The amounts of decellularized tissue, a graft, and a medicament used in the treatment or prophylactic method of the present invention can be easily determined by those skilled in the art, by considering the purposes of use, target diseases (type, severity, etc.), patient's age, weight, sex, case history, the form or type of a physiologically active substance, the form or type of the tissue, or the like.

The frequency of the method of the present invention being applied to a subject (or a patient) can be easily determined by those skilled in the art by considering the doses of the decellularized tissue, a graft, a pharmaceutical and a medicament, purposes of use, targeted diseases (type, severity, etc.), patient's age, weight, sex, case history, the course of therapy, and the like. The frequency includes, for example, once per day to once per several months (e.g. , once per week to once permonth). Preferably, administration is carried out once per week to once per month while observing progress.

As used herein, molecular biological techniques, biochemical techniques, and microbiological techniques well known in the art are optionally used. These methods are described in, for example, Ausubel F.A., et al., editors (1988), "Current Protocols in Molecular Biology", Wiley, New York, NY; Sambrook J. , et al. (1987), "Molecular Cloning: A Laboratory Manual" , 2nd Ed. , Cold Spring Harbor Laboratory Press, Cold Spring Harbor, NY; Bessatsu Jikken Igaku, "Idenshi-Donyu & Hatsugen-Kaiseki-Jikkenho" [Experimantal Medicine, Special Issue, "Experimental Methods for Gene Introduction & Expression Analysis", Yodo-sha, 1997; and the like.

In another aspect, the decellularized tissue, graft, and/or medicament of the present invention comprise instructions which provide guidelines for administration of the decellularized tissue, graft and/or medicament. Here, the instructions describe an appropriate method for administrating the decellularized tissue, graft and/or medicament. The instructions are prepared in a format defined by an authority in a country in which the present invention is used (e.g., the Health, Labor and Welfare Ministry in Japan, Food and Drug Administration (FDA) in the USA, etc.). The instructions explicitly describe the approval by the authority. The instructions are a so-called a package insert and are usually provided on a paper medium. The instructions are not limited to this. For example, the instructions may be provided in the form of an electronic medium (e.g., a web site on the Interwet, an electronic mail, etc.).

The decellularized tissue, tissue graft, and medicament of the present invention can be implanted using a technique well-known in the art (for surgery, see Hyojun-Geka-Kagaku [Standard Surgical Science], 9th ver. (Igakugakuin), Kihonteki-Geka-Shujutsu-Shugi [Basic Surgical Techniques] (pp. 41-66), Shinzo [Heart] (pp. 349-398), Kekkan [Blood Vessel] (pp. 399-428), and the like). The decellularized tissue of the present invention may be used in vascular anastomosis, cardiovascular reconstruction, vascular prosthesis replacement, prosthetic valve replacement, and the like. Therefore, those skilled in the art can apply the decellularized tissue, tissue graft, and medicament of the present invention in accordance with the disclosure of the present specification depending on circumstances where treatment is performed.

In the present invention, the medicament of the present invention may be applied to any part of the body of an organism. Therefore, the tissue graft may be applied to a part of the body from which the medicament was derived or other parts of the body. As shown in the Examples, it was demonstrated that the desired effect (e.g., regeneration, self organization) of the present invention can be achieved for any part of the body to which the medicament of the present invention is applied, no matter whether or not the part is a site from which the tissue was derived. Therefore, the present invention has a considerably advantageous utility such that the present invention can in principle be applied to any implantation operation and regeneration operation. Examples of parts to which the medicament of the present invention is applied, include, but are not limited to, skin, blood vessel, cornea, kidney, heart, liver, umbilical cord, intestine, nerve, lung, placenta, pancreas, brain, peripheral limb, retina, valve, epithelial tissue, connective tissue, muscular tissue, nervous tissue, and the like.

Hereinafter, the present invention will be described by way of examples. The following examples are provided only for illustrative purposes. Therefore, the scope of the present invention is limited only by the claims.

EXAMPLES

Hereinafter, the present invention will be described in greater detail by way of examples. The present invention is not limited to the examples below. Reagents and the like used in the following examples are commercially available from Sigma (St. Louis, USA), Wako Pure Chemical Industries, Ltd. (Osaka, Japan), and the like, with exceptions. Animal experiments were conducted in accordance with the guidelines established by Osaka University.

(Example 1)

(Materials and Methods)

(Decellularization by PEG)

Porcine carotid arteries were prepared from Hybrid (Labo Products Co. Ltd. , Osaka, Japan), and rat aortas were prepared from SD rats (male, 5 weeks old, Nippon Animal Co., Ltd., Tokyo, Japan) under sterile conditions. Animal experiments were conducted in accordance with the guidelines for ethics established by Osaka University.

Freshly collected porcine carotid arteries and rat aortas were placed in PBS (referred to as PBS (-) in this example; Gibco BRL, Life Technologies Inc. Rockville, MD, USA) containing antibiotics (Gibco BRL, Life Technologies Inc. Rockville, MD, USA) to wash out blood components. The blood vessels were then placed in a decellularizing aqueous solution containing polyethylene glycol (1g/ml, Nacalai Tesque Inc., Kyoto, Japan) (average molecular weight: 1000), and allowed to stand for 0.5h. Because of high viscosity of the solution, the blood vessels were gently pressed several times with a glass rod at room temperature. The blood vessels were placed in PBS (-) containing antibiotics (100 units of penicillin, 0.1mg of streptomycin, 0.25$\mu$g/ml amphotericin B; all of which are available from Gibco BRL, Life Technologies Inc. Rockville, MD, USA) on a rotor (Tube rotator TR-118: Iuchi Co. Ltd, Osaka, Japan) at room temperature. The wash solution was changed every 24 hours over 72 hours. After rinsing, the blood vessels were immersed in PBS (+) (PBS (-) supplemented with 5 mM MgCl$_2$) containing DNaseI (Takara Shuzo Co., Ltd., Shiga, Japan) at 37°C for 1 h. The blood vessels were placed in the above-described PBS (-) containing antibiotics on a rotor at room temperature. The wash solution was changed every 24 hours over 72 hours. After rinsing, the blood vessels were preserved in PBS (-) containing antibiotics at 4°C.

When using PEGs having average molecular weight of 1,000, 2,000, 200 and 6,000, those having any molecular weight may be used. It seemed that PEGs having average molecular weight of 2000 and 6000 achieved more a satisfactory decellularization treatment, though PEG having an average molecular weight of 1000 was more easily handled in decellularization treatment.

Next, decellularized tissue was prepared in accordance with another decellularization procedure. The procedure will be described below.

(Decellularization method by SDS, No. 1)

1) (Washing)

Tissue was washed with physiological saline for one hour at 4°C.

2) (First surfactant treatment)

The tissue was placed in physiological saline containing 1% SDS (sodium dodecyl sulfate, SIGMA L-4509) and allowed to stand at room temperature for 48 hours.

3) (Washing)

The tissue was removed and was placed again in physiological saline and allowed to stand for 24 hours at room temperature.

4) (Second surfactant treatment)

The tissue was placed in physiological saline containing 1% NP-40 (SIGMA I-3021) and was allowed to stand at room temperature for 48 hours.

5) (Washing)

The tissue was removed and was placed again in physiological saline and allowed to stand for 24 hours at room temperature.

6) (Sterilization)

The tissue was placed in 20% isopropanol (SIGMA I-0398) and was aseptically preserved before use or experiment.

(Decellularization method by SDS, No. 2)

1) (Washing)

Tissue was placed in physiological saline containing a protease inhibitor (PROTEASE INHIBITOR COCKTAIL; SIGMA P2714) and 20mM EDTA and washed for 24 hours at 37°C.

2) (First surfactant treatment)

The tissue was placed in physiological saline containing 1% SDS (sodium dodecyl sulfate, SIGMA L-4509) and was allowed to stand at room temperature for 72 hours.

3) (Washing)

The tissue was removed and was placed in physiological saline and allowed to stand for 48 hours or more at 37°C.

4) (Second surfactant treatment) The tissue was placed in physiological saline containing 1% NP-40 (SIGMA I-3021) and was allowed to stand at room temperature for 48 hours or more.

5) (Washing)

The tissue was removed and was placed again in physiological saline for 48 hours at 37°C.

6) (Cryochemistry process)

A cryochemistry process was performed in accordance with a commonly used method.

7) (Sterilization)

The tissue was placed in 0.05% sodium azide and was aseptically preserved before use or experiment.
The above-described procedure was performed in accordance with conventional methods.

(Triton treatment)

1) (Washing)

Tissue was washed with physiological saline for 1 hour at 4°C.

2) (Surfactant treatment)

The tissue (e.g., a blood vessel)was immersed in physiological saline containing 1% Triton (registered trademark) X-100 (ICN Biomedicals, Inc., CA, USA) +0.1% ammonium hydroxide (Wako Pure Chemical Industries Ltd., Osaka, Japan), i.e., a decellularization solution. The decellularization solution was changed every 24 hours using a shaker at 4°C.

3) (Washing)

Thereafter, the tissue was washed by changing PBS every 24 hours using a shaker at 4°C a total of three times.

(Decellularization method by SDS, No. 3)

1) Tissue wash: 0.3% NaCl, protease inhibitor (PROTEASE INHIBITOR COCKTAIL; SIGMA P2714), 20 mM EDTA, 20ºC, 24 hrs
2) Treatment with detergent: 0.5% SDS (sodium dodecyl sulfate, Sodium Laurel Sulfate, SIGMA L-4509)+ 0.5% Triton X-100(ICN Biomedicals Inc.CA.USA) in 0.3% NaCl + 0.05% $NaN_3$, room temperature
3) Wash: 0.3% physiological saline + 0.05% $NaN_3$, room temperature, at least 120 hours
4) Sterilization and preservation: cold chemical treatment: place the sample for 3 hours, and soak in a saline solution containing 0.03% $NaN_3$.

Accordingly, decellularized tissue using such amphipathic solution may be subjected to treatments with any biocompatible macromolecules as described below.

(Treatment of biocompatible macromolecule)

Polyvinyl alcohol solution prepared at an appropriate concentration such as about 1%-30%m preferably about 10%, was coated onto the outer surface of decellularized tissue (decellularized cardiac valve or the like) by means of brush or brushes or the like. Thereafter, the sample was placed in a glass sampling vessel (size: diameter about 5cm x height about 8cm) with a plastic cap, and gamma-ray irradiation was conducted using Cobalt 60 irradiation source in the state of closing the cap (tissue and air existed inside). Temperature was room temperature and the temperature rose as the irradiation progressed by the heat caused thereby, and in irradiation it was between 25°C and 40°C. Washing may or may not be performed after decellularization and before gamma-ray irradiation.

(Collagen structure)

It has been revealed that collagen structure is affected by pH, ionic strength, the polarity of a solvent, anionic surfactants, and the like (Ripamonti A., et al., 1980, Biopolymers 19:965-975; and Xiao W. H. , Knight D. P. , Chapman J. A., 1997, Biochimica et Biophysica Acta. 1134:327-337). It is also known that a change in collagen structure results in an alteration in bioengineering characteristics. In order to avoid these problems, a decellularization procedure was designed in this example so that matrices are not affected. It was demonstrated by histological tests and tensile strength measurements of the collagen structure that matrices were absolutely not impaired as follows.

(Histological examination)

Paraffin sections (thickness: 3μm) of a blood vessel graft were prepared and stained with hematoxylin-eosin to identify extracellular matrices. To identify I/IV collagen which is a component of the basal membrane, immunohistochemical staining was used. Aortas of SD rats (male, 5 weeks old, Nippon Animal Co., Ltd., Tokyo, Japan) were fixed with 4% paraformaldehyde and cryoprotected. Frozen sections (thickness: 5μm) were prepared, followed by permeabilization with PBS (-) for 3h and then blocking with 1% BSA in PBS (-) for 1h at room temperature. Subsequently, the sections were incubated with primary antibodies (anti-rat collagen antibodies, Cosmo Bio, Tokyo, Japan), and then with secondary antibodies conjugated with FITC (anti-sheep Ig antibodies; Cosmo Bio, Tokyo, Japan). Images were obtained with a Zeiss LSM 510 confocal microscope.
von Kossa staining was conducted as follows: optionally deparaffin treatment (for example, with pure ethanol), wash in water (distilled water) are conducted and immerse the sample in 25% silver nitrate (under indirect light) for two hours. Thereafter, wash with distilled water, and immerse the sample in 42% sodium thiosulfate (hypo) for five minutes. Thereafter, it was washed in running water for five minutes, and then nucleus fast Red for 5min. The sample is then washed with running water for 5 min, followed by dehydration, clearing, and mounting.

(Results)

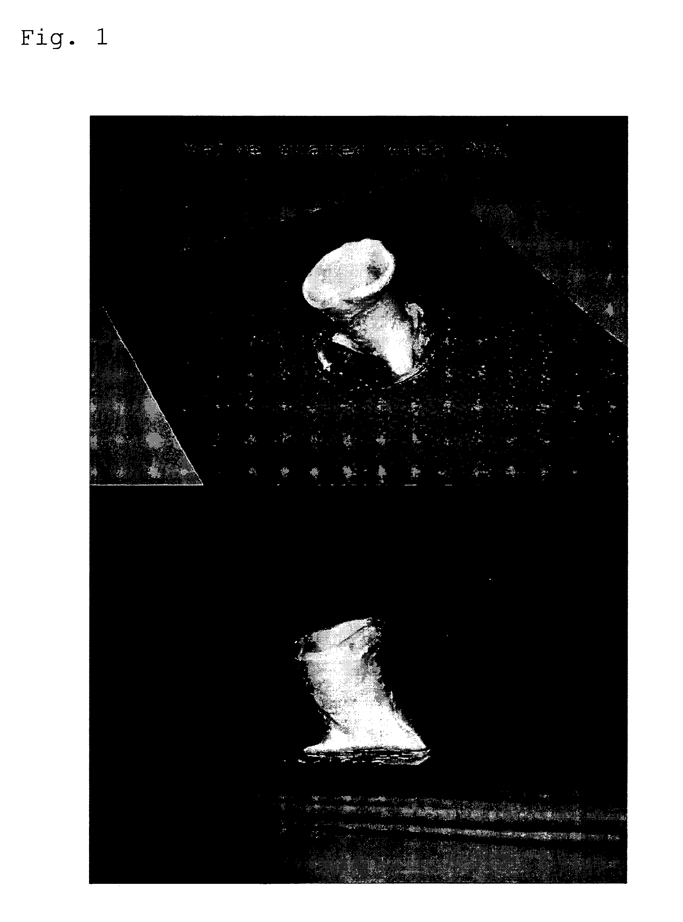

Cytoplasmic components of the native tissue has been removed to some extent by means of the decellularization treatment of the present Example. Figure 1 shows the results thereof.

(Cell redisual rate)

A cell redisual rate was calculated by counting the number of nuclei in an area of $100\mu m \times 100\mu m$ using a microscope. Specifically, the number of nuclei in the same sample was counted in the area before and after treatment. The number of nuclei in the sample after treatment was divided by the number of nuclei in the sample before treatment and the result was multiplied by 100 to obtain a cell redisual rate (%). The results are shown in Table 1.

(Tissue injury rate)

A visual field was divided into units of $100\mu m \times 100\mu m$ and the number of units having an elastin rupture site were counted. There were 24 units per visual field. Elastin rupture was represented by x/24. x was counted when rupture was found in a unit. For example, when no rupture was confirmed (e.g., untreated control), the damage rate was calculated as 0/24, i.e., 0%. The results are summarized in Table1.

Table 1

|  | Cell redisual rate | Tissue damage rate |
|---|---|---|
| No treatment | 100% | 0% |
| PEG treatment | 4.7% | 15% |
| TRITON treatment | 17.8% | 34% |
| SDS treatment | 3.0% | 30% |
| PEG treatment + PVA | 4.7% | 15% |
| SDS treatment +PVA | 3.0% | 30% |
| 1) calculated by an average of the number of nuclei in the unit area of $100\mu m$ x $100\mu m$ <br> 2) One visual field is divided per $100\mu m$ x $100\mu m$, and the number of units having an elastin rupture site was counted (24 units per visual field). | | |

From the results above, it has been demonstrated that gamma-ray irradiation to the biocompatible macromolecule reduced the cell redisual rate. Cell redisual rate was observed by a counting method using a microscope with hematoxylin and eosin staining (H&E staining). Hematoxylin and eosin staining procedures are as follows: Optionally, deparaffin treatment (for example, with pure ethanol) was performed and water wash was performed, and the sample was soaked in hematoxylin from Omni for ten minutes. Thereafter, washing was performed with running water, and ammonia water was used for developing color for 30sec. Thereafter, washing was performed with running water for 5min, staining was performed for 2 min using eosin hydrochloride ten-fold diluted solution, and dehydrated, lucidification was performed and mounted.

(Tissue strength)

Tension strength, elasticity and extension were observed by tension test. The general outline therefor is described hereinbelow:
The present Example measured the strength by tension testing device (TENSILLON available from ORIENTEC). Specifically, tas used herein, (1) specimen is cut in a state of 5 x 30 mm rectangular form (basically, specimen is cut such that wall portion of aortic basal portion becomes longer in the longitude direction); (2) fixation portion of tension testing device is fixed for about 5 mm at both ends of the specimen (ORIENTEC, TENSILON using universal testing device RTC-1150A); and (3) start streching and continue streching at a rate of 1cm/min to the rupture portion, which were used for measuring rupture load and elasticity (Shinoka T, Mayer JE. Tissue engineering heart valve leaflets.Circulation.1997; 96 (suppl. II): II-102-II-107; Shum-Tim D, Mayer JE. Tissue engineering of autologus aorta using a new biodegradable polymer. Ann Thorac Surg. 1999; 68: 2298-2304).

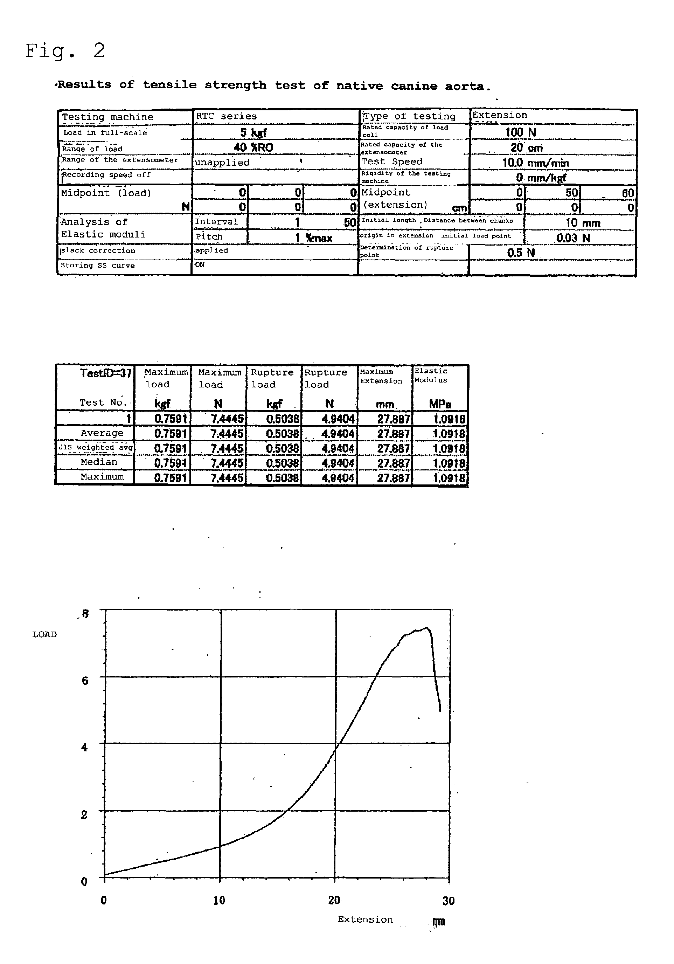

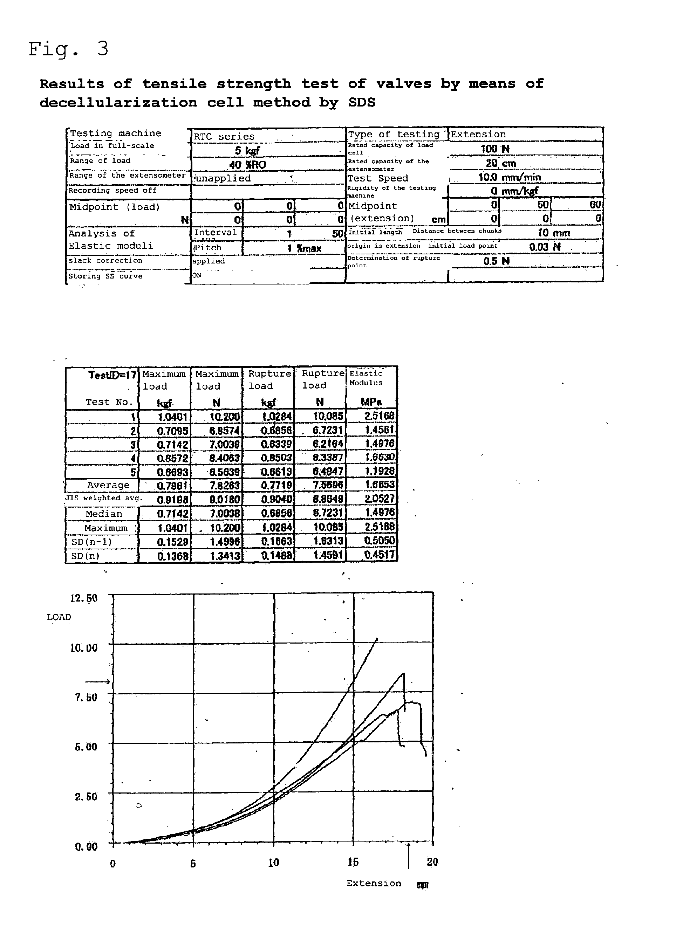

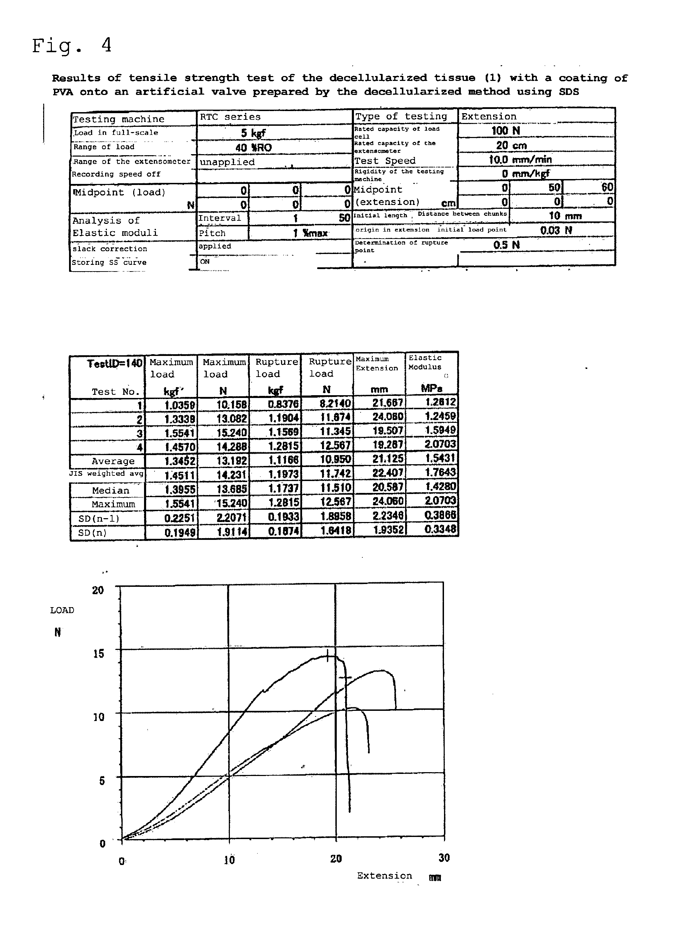

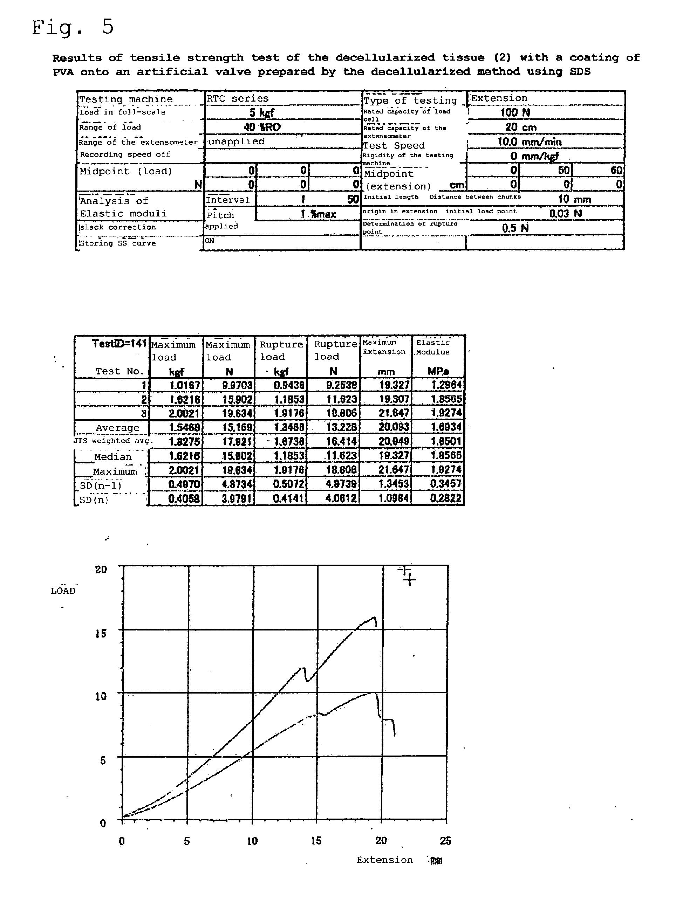

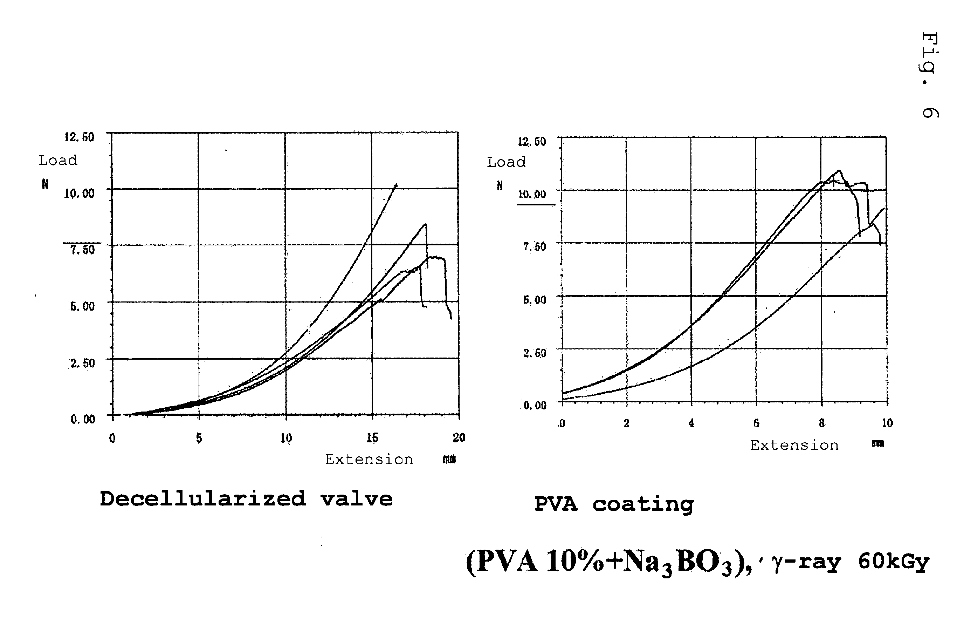

The results thereof are shown in Figures 2-6 and Table 2.

TABLE 2

| | Maximum point load (N) | Maximum point extension (mm) | Elasticity (MPa) |
|---|---|---|---|
| Living Aorta (canine) | 7.4 | 28 | 1.1 |
| SDS (Decellularization method by SDS No. 3) | '7.8 | 18 | 1.7 |
| Valve (Decellularization method by SDS No. 3 + PVA + gamma ray irradiation(60kGy)) | 14 | 21 | 1.6 |

A biocompatible macromolecule (PVA) was coated onto such a decellularized tissue, and gamma-ray irradiation was subjected thereto to show the unexpectedly significant improvement in tissue strength. As shown in Table 1, tissue strength was reinforced to two fold, as compared to that of native valve and those before treatment . Such an effect of the biocompatible macromolecule has not been known and thus should be recognized to be a significant effect.

The decellularized tissues according to the present invention maintain properties so as to be able to use in terms of extension and elasticity (see Figures 2-6 and Table 2).

As described above, it was revealed that biocompatible macromolecule treatment according to the present invention achieved tissue strength higher than that had been achieved by conventional decellularized tissues.

(Example 2: Comparison of reactions within biological tissue)

(Method)

(Immunological response)

Aorta wall portions ($1 \times 1$cm) of a gamma-ray and decellularization treated valve according to Example 1 and an SDS-treated decellularization tissue (the artificial valve prepared by SDS decellularization No. 3) according to Example 1 were implanted under the skin of the dorsal portions of SD rats. After one week and two months, the animals were sacrificed. The degree of inflammatory cellular infiltration was scored for evaluation. In this example, porcine native valves were used as controls for comparison.

(Calcification)

The specimens were collected one week and two months after subcutaneous implantation, followed by von Kossa staining for evaluation of calcification. Also, Ca concentration within the tissue was measured with an atomic absorption spectrometry. The Ca concentration was measured and quantified as follows. The tissue was placed in concentrated hydrochloric acid (or concentrated acid) and was dissolved while heating. Thereafter, atomic absorption spectrometry was performed. The solution was diluted and sprayed into high temperature plasma. Element-specific absorption wavelengths of spectra generated in combustion were measured (Ca: 393.366 nm).

Aortic wall portions ($10 \times 10$mm) of decellularization-treated porcine aortic valves were implanted under the skins of the dorsal portions of SD rats (250g). After two months, the specimens were collected. A Ca concentration (Ca content per dry weight) within the tissue was measured with an atomic absorption spectrometry (SPS7800: Seiko Instrument Inc.) (see, Ozaki S., "Pathophysiology of Calcification of Bioprosthetic Heart Valves", Acta Biomedical Lovaniensia, 2001).

(Implantation)

A portion of aortic wall tissue treated by the first generation decellularization process was implanted to dog descending aortas.

(Results)

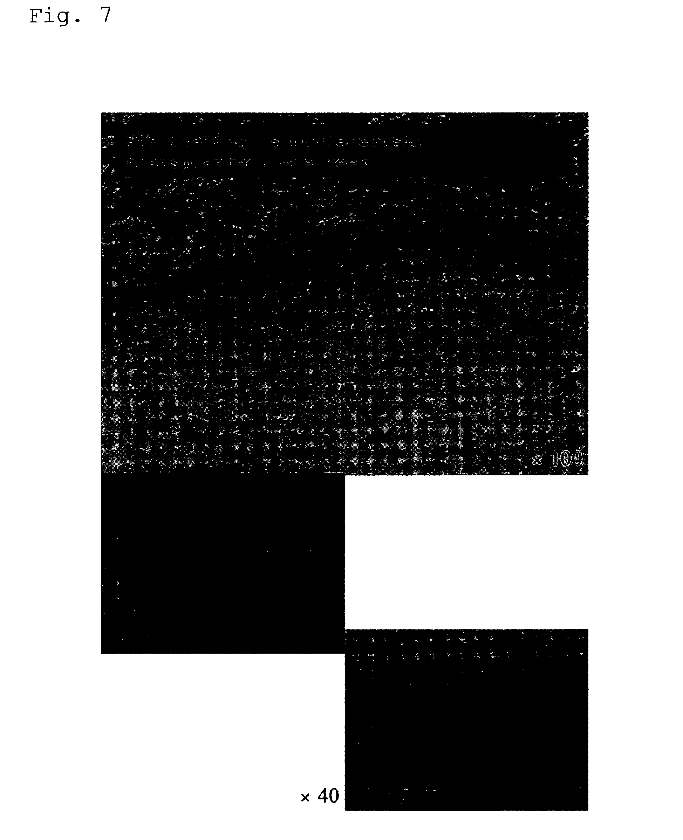

Results of one week after the subcutaneous implantation are shown in Figure 7. As seen from Figure 7, it was found that: no inflammatory cellular infiltration was observed. On the other hand, inflammatory cellular were observed in a plurality of layers in the porcine native valve; and in the SDS-treated valve, substantially no cellular infiltration was observed, only a slight inflammatory reaction was observed, and the whole tissue structure was not impaired.

(Example 3: Confirmation of cell replacement)

Comparison of Reactions in Biological Tissue between each Valve. Decellularization-treated porcine forearm arteries according to Example 1 are implanted into dog femoral aortas. The animals are sacrificed after 10 days. The degree of inflammatory cellular infiltration is compared and studied.

(Results)

It is found that there is substantially no inflammatory reaction in the decellularized tissue of the present invention, and thus it is understood that global structure is not impaired. Further, by observing the implanted tissue, it is also possible to confirm that the decellularized tissue is replaced with self cells.

(Example 4: Decellularization of pericardia)

Next, pericardia was removed from swine and treated with a surfactant solution or a polyethylene glycol solution as described in Example 1. The cell redisual rate and tissue damage rate of the treated pericardia are measured. As a result, decellularized pericardia having a tissue damage rate of less than 30% and a cell redisual rate of less than 10% are obtained.

The pericardia are implanted to SD rats as described in Example 2 and are examined for inflammatory cellular infiltration and calcification. Decellularized tissue and control tissue are implanted to 3-week old rats, followed by monitoring for 60 days. After excision of the tissue, samples are collected for histological evaluation. The samples are stained by hematoxylin-eosin (H&E) staining for evaluation of the whole structure.

Next, this decellularized pericardium was implanted into rat cardiac infarct.

(Cardiac infarct rat model)

Lewis male rats are used in this example. The animals are cared for in compliance with the spirit of animal protection in accordance with "Principles of Laboratory Animal Care" prepared by National Society for Medical Research and "Guide for the Care and Use of Laboratory Animals" (NIH Publication No.86-23, 1985 revised) prepared by Institute of Laboratory Animal Resource and published by National Institute of Health.

Acute cardiac infarction is induced as described in Weisman H. F., Bush D. E., Mannisi J. A., et al. "Cellular mechanism of myocardial infarct expansion", Circulation, 1988;78:186-201. Briefly, rats (300 g, 8 weeks old) are anesthetized with pentobarbital, followed by positive pressure breathing. In order to produce rat cardiac infarction models, the chest is opened via the left 4th intercostals and the left coronary artery is ligated at a distance of 3mm from the root thereof with 8-0 polypropylene thread.

(Implantation)

Recipient rats are anesthetized. The chest is opened via the left 5th intercostals to expose the heart. The rats are divided into two groups, depending on the substance which is administered into a cardiac infarct region of the rat, i.e, group C (no treatment, n=5) and group S (implanted with decellularized pericardium tissue, n=5). The decellularized pericardium is directly implanted into the infarct site two weeks after ligation of the left anterior descending branch.

(Measurement of function of rat heart)

Heart functions are measured by echocardiography (SONOS 5500, produced by Agilent Technologies) 2 weeks, 4 weeks, and 8 weeks after production of the infarction model. Minor axis images are drawn at positions where the left ventricle has the greatest diameter when viewed from the left, using a 12-MHz transducer. In the B-mode, the end systolic area of the left ventricle is measured. In the M-mode, the end diastolic diameter of the left ventricle (LVDd), the end systolic diameter of the left ventricle (LVDs), and the anterior wall thickness of the left ventricle (LVAWTh) are measured to obtain LVEF and LVFS.

(Histological analysis)

Eight weeks after implantation, the heart is removed. The heart is cut along a minor axis. The heart is immersed in 10% formaldehyde solution, followed by paraffin fixation. Sections are prepared, followed by hematoxylin-eosin staining and Masson trichrome staining. At the same time, the sections are frozen, followed by factor VIII immunological staining.

Thus, it is demonstrated that the decellularized tissue of the present invention can be applicable to sites other than a site from which tissue is derived before treatment .

(Example 5: Decellularization of Dura matter)

Next, dura matter is removed from swine and were treated with a surfactant or a polyethylene glycol solution as described in Example 1. The cell redisual rate and tissue damage rate of the treated dura matter are measured. As a result, decellularized dura matter having a tissue damage rate of less than 30% and a cell redisual rate of less than 5% are obtained.

The dura matters are implanted to Lewis rats as described in Example 2 and are examined for inflammatory cellular infiltration and calcification.

Thus, according to the method of the present invention, any tissue can be treated into decellularized tissue having similar superior characteristics.

(Example 6)

Next, as other biocompatible macromolecules, polyvinyl pyrrolidone, gamma-polygiutamic acid, gelatin other than polyvinyl alcohol are tested for the same experiments as in Example 1. These biocompatible macromolecules will be crosslinked and gellified in conditions of the same gamma-ray irradiation (concentration 10%, same glass vessel, and about 60-100kGy irradiation using Cobalt 60 irradiation source). Therefore, by conducting similar experiments as in Example 1, it is possible to confirm reenforcement effects on decellularized tissues in a similar manner. Specifically, maximum point load (N), maximum point extension (mm), elasticity (MPa) are measured as in Example 1, to confirm similar reinforcement effecst by using polyvinyl pyrrolidone, gamma-polyglutamic acid, gelatin in addition to polyvinylalcohol.

(Example 7)

Next, whether or not similar effects can be expressed in the decellularized tissue of blood vessel, intestinal tract, pericardium, cornea, retina, bone, and ureter. Similar decellularization experiments are conducted as in Example 1. These have been crosslinked and gellified by using polyvinyl alocohol, polyvinyl pyrrolidone, gamma-polyglutamic acid and gelatin. Fox the enhanced decellularized tissues, similar experiments are conducted as in Examples 2-5, which allows confirmatino of enhancement effects of decellularized tissues. Specifically, maximum point load (N), maximum point extension (mm), elasticity (MPa), are measured as described in Example 1, to confirm that decellularized tissues other than valve such as blood vessel, intestinal tract, pericardium, cornea, retina, bone, and ureter can be similarly reinforced.

(Example 8: Investigation of optimum parameters)

Next, a variety of treatment methods are used to produce decellularized tissues, which were studied for the effects of reinforcement by means of PVP, PVA and PEG. The protocols and results thereof are described herein below:

A. Decellularization treatment by means of SDS, TritonX-100

(i) Reagents:

a. Decellularization solution
0.5%SDS, 0.5%TritonX-100, 0.3%NaCl, 0.1%NaN3, 100U/ml penicillin, 100$\mu$g/ml streptyomycein (GIBCO), 1.25$\mu$g/ml amphotericin B(SIGMA), 100mg/l kanamycin(SIGMA)

b. washing solution
(prepared in PBS(-)(NaCl 8.0g, KCl 0.2g, Na$_2$HPO$_4$ 1.15g (in case of Na$_2$HPO$_4$·12H$_2$O 2.9g), KH$_2$PO$_4$ 0.2g (per 1,000ml, pH=7.2-7.4)))
20mM EDTA, Protease inhibitors cocktail, 0.1%NaN$_3$, 100U/ml penicillin, 100$\mu$g/ml streptomycin (GIBCO),

1.25μg/ml amphotericin B (SIGMA), kanamycin 100mg/l (SIGMA)

c. sterilization solution
(prepared in PBS(-)
(20mM EDTA, 1%IPA, 100U/ml penicillin, 100μg/ml streptomycin (GIBCO), 1.25μg/ml amphotericin B (SIGMA), 100mg/l kanamycin (SIGMA)

d. storage solution
(prepared in PBS(-))
20mM EDTA, 10mM HEPES, 0.1%$NaN_3$, 100U/ml penicillin, 100μg/ml streptomycin (GIBCO), 1.25μg/ml amphotericin B (SIGMA), 100mg/l kanamycin (SIGMA)

e. rinse solution
0.1%$NaN_3$, 100U/ml penicillin, 100μg/ml streptomycin (GIBCO), 1.25μg/ml amphotericin B (SIGMA), 100mg/l kanamycin (SIGMA)

(ii) steps:

(1) The tissue was immersed in decellularization solution (500ml) for 24 hours, at 25°C, at 200 revolutions/min. Rotation was conducted in Bio-Shaker BR-300LF (TAITEC).
(2) Fresh decellularization solution (500ml) was replaced therewith, and continued immersion for 24 hours, at 25° C, at 200 revolutions/min. Rotation was conducted in Bio-Shaker BR-300LF (TAITEC).
(3) Fresh decellularization solution (500ml) was replaced therewith, and continued immersion for 24 hours, at 25°C, at 200 revolutions/min. Rotation was conducted in Bio-Shaker BR-300LF (TAITEC).
(4) The tissue was immersed in a rinse solution for 12 hours, at 25° C, at 200 revolutions/min. Rotation was conducted in Bio-Shaker BR-300LF (TAITEC).
(5) Fresh rinse solution (500ml) was replaced therewith, and continued immersion for 24 hours, at 25° C, at 200 revolutions/min. Rotation was conducted in Bio-Shaker BR-300LF (TAITEC).
(6) The tissue was immersed in sterilization solution for three hours, at 25°C, at 200 revolutions/min. Rotation was conducted in Bio-Shaker BR-300LF -(TAITEC).
(7) (if storage was performed) the tissue was immersed in a storage solution, and was stored at 4°C.

B. Prosthetic decellularized tissue by PVP coating and gamma-ray irradiation

(i) Reagents
a. PVP solution
PVP (polyvinylpyrrolidone) [average molecular weight 40,000] <SIGMA>
10 w/w% solution was prepared.

(ii) steps::

(1) The tissue was immersed in PVP solution (10 w/w%) and removed thereafter.
(2) gamma-ray (15kGy or 60kGy) were radiated.

The following conditions were used to investigate parameters for reinforcement experiments of decellularized tissue.

(PVP: 15kGy)     (i)-a
(PVP: 60kGy)     (i)-b
(PVA: 15kGy)     (ro)-a
(PVA: 60kGy)     (ro)-b
(PEG: 15kGy)     (ha)-a
(PEG: 60kGy)     (ha)-b

To the bovine pericardium treated with decellularization treatment as described below in (1) through (3), the present prosthetic methods (i)-a through (ha)-b were applied, the effects "before and after the application, evaluation indexes A-C (A: tension strength; B: elasticity; and C: how much extent the presence or absence of rejection response two weeks after transplantation into rat (T cells or like, presence or absence of infiltration into cells relating to rejection response) is improved.) " was used for evaluation.

<Decellualization method>

(1) SDS, triton X-100 treatment (prior art) -> tensile strength of the treated tissue was reduced and has properties of inducing rejection reaction.
(2) PEG+gamma-ray (15kGy)+DNase treatment -> tensile strength of the treated tissue and elasticity were well conserved and Inducibility of rejection reaction was reduced.
(3) PEG + gamma-ray (100kGy)+DNase treatment -> tensile strength of the treated tissue and elasticity were well conserved and inducibility of rejection reaction was reduced.

(Results)

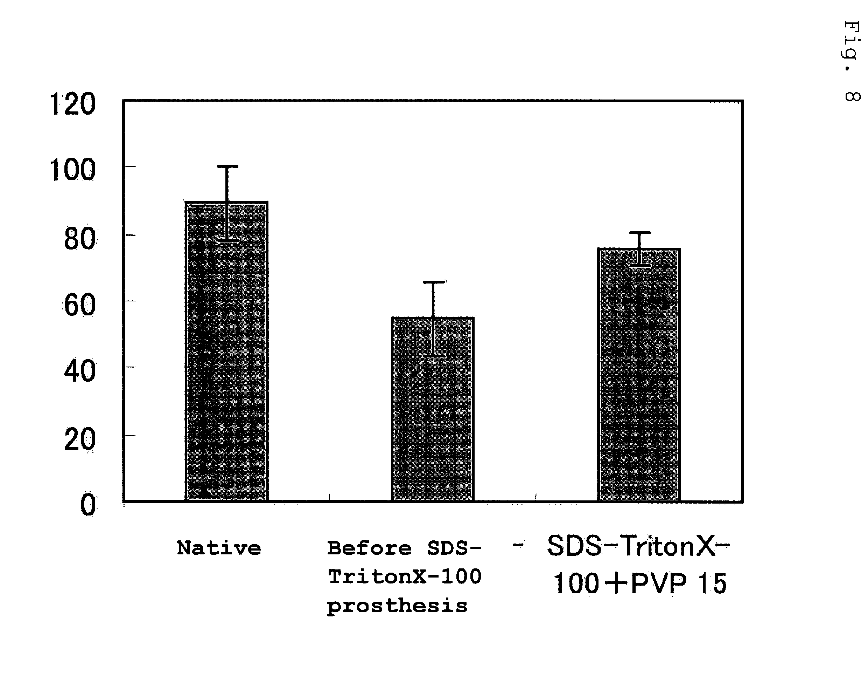

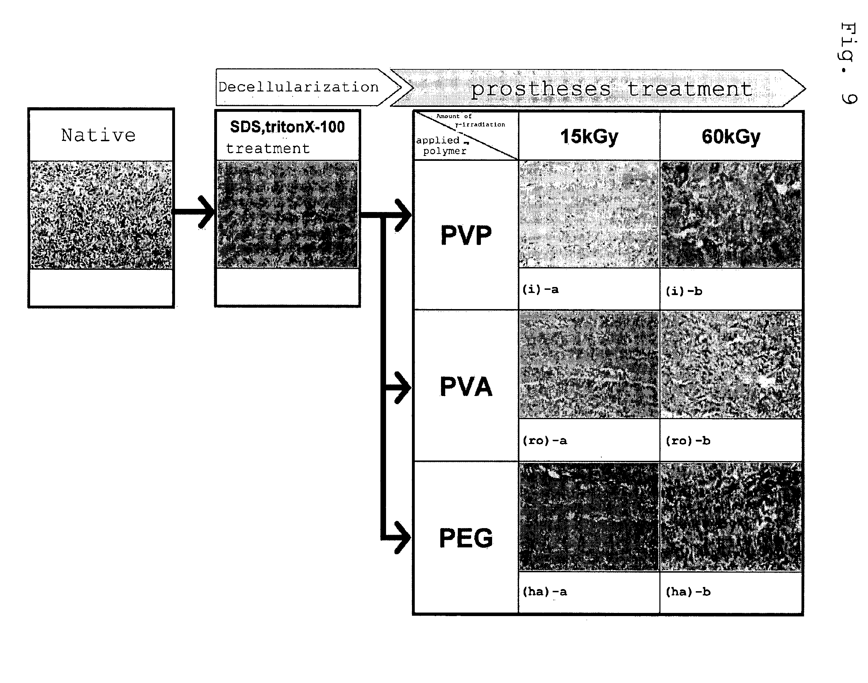

Decellularization treatment (1) has improved strength by means of method (i)-a with respect to parameters for tensile strength (Figure 8). As described in Figure 8, the tissue treated with a method of decellularization (SDS-Triton X-100 treatment) having difficulty in reduction of tension strength, was subjected to the application of the inventive prosthetic method (PVP coating -> gamma-ray 15kGy irradiation), to result in improvement of tension strength. Preventive effects against the rejection response were observed in the subject treatment. The present effects were observed in all the methods of (i)-a through (ha)-b, in that cell infiltration relating to rejection response was prevented (Figure 9).
Figure 9 shows results of prosthetic treatment of SDS, Triton X-100 treated membrane (evaluation by means of rat dorsal transplantation). In Figure 9, histological photographs are shown for (1) transplantation site: rat dorsal site; (2) two weeks after the transplantation; (3) HE staining; and (4) magnification: x 100 for the experiments. Naive tissue was observed to have significant infiltration to cells relating to rejection reaction (T cells or the like). In SDS, Triton X-100 treatment alone (only decellularized treatment), infiltration of cells relating to the rejection response was slightly observed. In all the treatment methods from (i)-a through (ha)-b, no infiltration to cells relating to rejection response were not observed.
Accordingly, the present prosthetic method is applied to tissues having properties raising rejection response has altered into tissues which do not raise rejection reaction (i.e. there was blocking effects of rejection response).

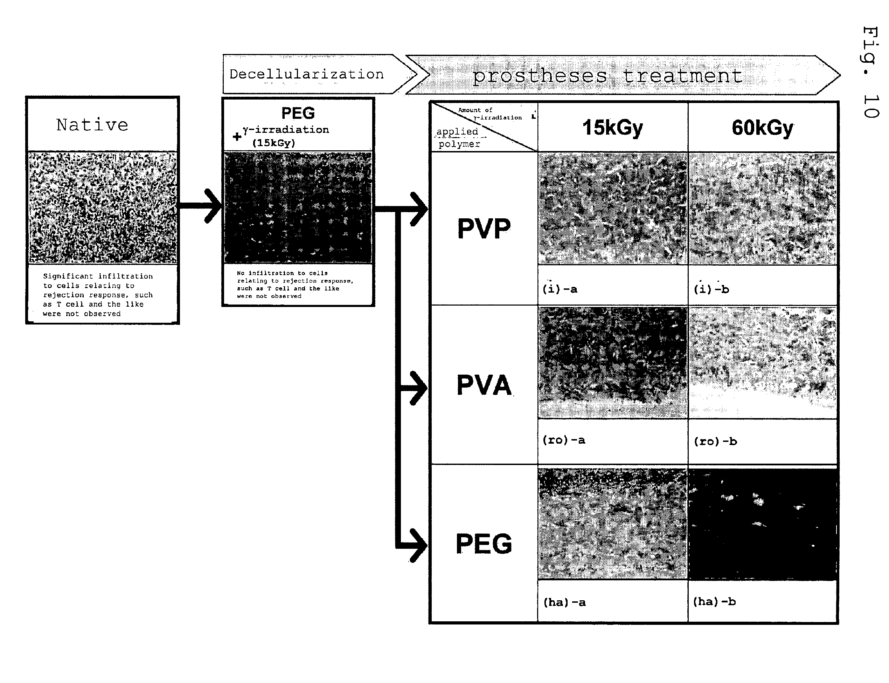

Figure 10 depicts uses a decellularized tissue prepared by decellularization treatment method No. (2) to confirm presence/absence of rejection reaction. Experimental conditions used in Figure 10 is gamma-ray 15kGy irradiation -> PEG -> DNase treatment for membrane prosthetic treatment (evaluation is made by means of rat dorsal transplantation). Experiments have been conducted using the following conditions: rat dorsal site; (2) two weeks after the transplantation; (3) HE staining; and (4) magnification: x 100. Naive tissue was observed to have significant infiltration to cells relating to rejection reaction (T cells or the like). In the tissue with PEG+gamma-ray irradiation (15kGy), in all the treatment methods from (i)-a through (ha)-b, no cell infiltration to cells relating to rejection response were not observed. Accordingly, even if the present prosthetic method was applied to tissues having properties not raising rejection response, no rejection response was raised (i.e. no rejection response was raised due to the prosthesis method). In decellularization method No. 2, tissue strength and elasticity were not lowered, and thus no effects were observed with respect to the reinforcement by the present invention.

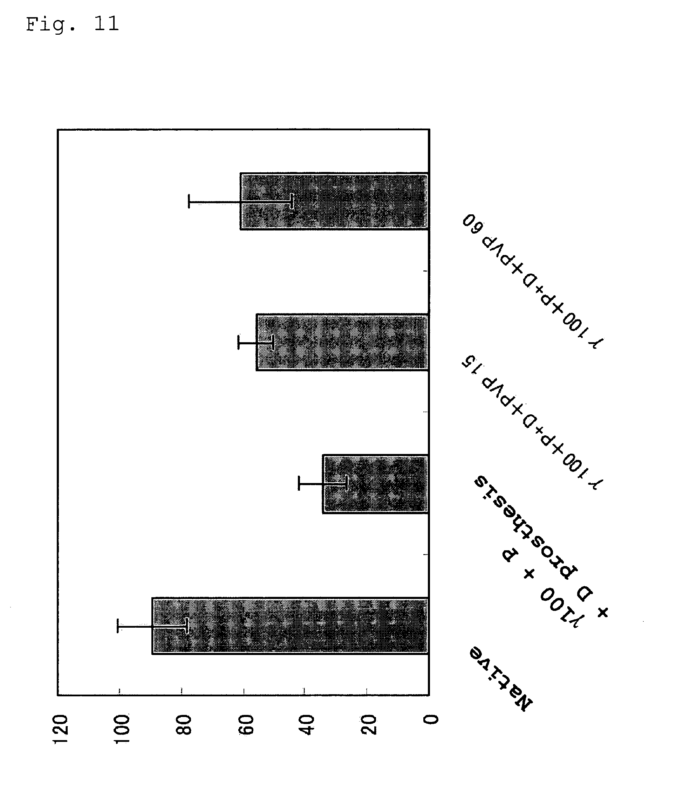

Figure 11 depicts the results of gamma-ray 100kGy irradiation -> PEG -> DNase treatment for membrane prosthetic treatment (evaluation is made based on tension strength). In the figure, the results obtained by treatment using PVP are shown. Tissues treated with a method for decellularization (gamma-ray 100kGy irradiation -> PEG -> DNase treatment) having difficulty in reduction of tension strength, was subjected to the present prosthesis method ("PVP coating -> gamma-ray 15kGy irradiation" and "PVP coating -> gamma-ray 60kGy irradiation"), to result in improvement in tension strength. As shown therein, treatment using PVP resulted in significant improvement in the strength thereof.

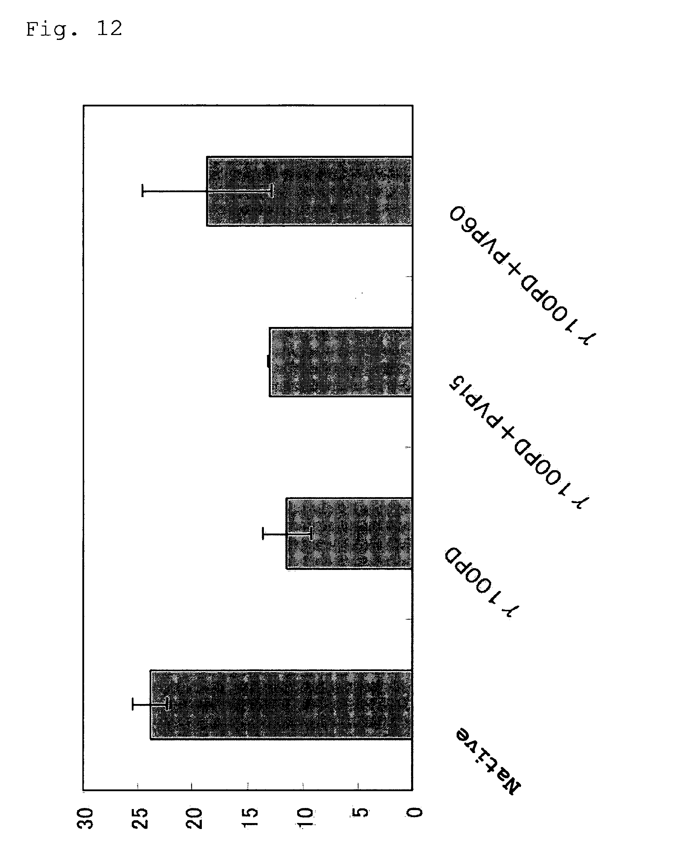

Figure 12 depicts the results of gamma-ray 100kGy irradiation -> PEG -> DNase treatment for membrane prosthetic treatment (evaluation is made based on elasticity). Tissues treated with a method of decellularization having difficulty in reduction of elasticity (gamma-ray 100kGy irradiation -> PEG -> DNase treatment) was subjected to the present prosthesis method ("PVP coating -> gamma-ray 15kGy irradiation" and "PVP coating -> gamma-ray 60kGy irradiation"), to result in improvement in elasticity.

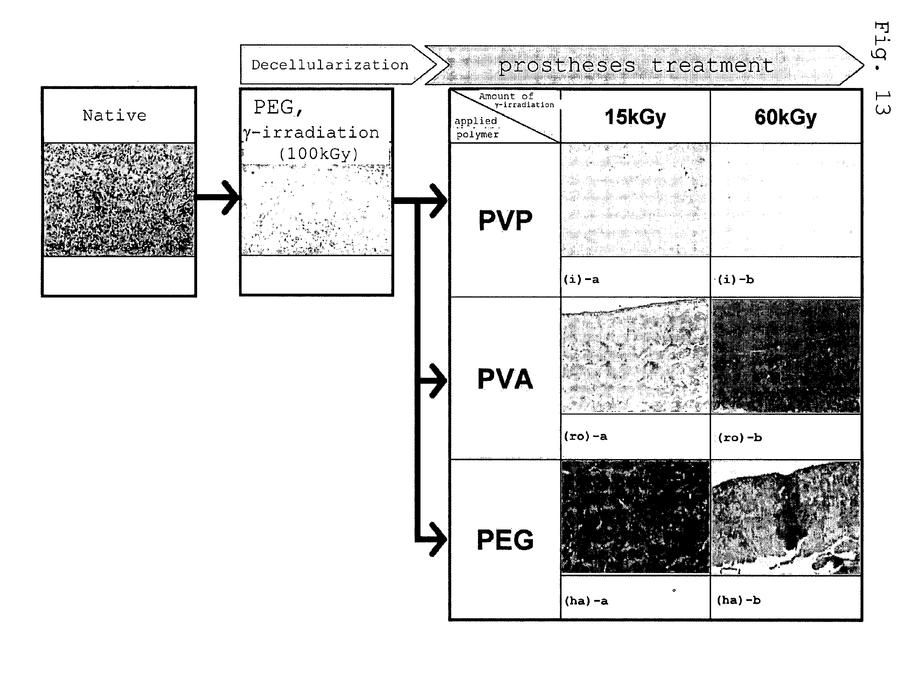

Figure 13 depicts the results of gamma-ray 100kGy irradiation -> PEG -> DNase treatment for membrane prosthetic treatment (evaluation is made by means of rat dorsal transplantation). Results are shown for (1) transplantation site: rat dorsal site; (2) two weeks after the transplantation; (3) HE staining; and (4) magnification: x 100 for the experiments. Naive tissue was observed to have significant infiltration to cells relating to rejection reaction (T cells or the like). Tissues treated with a method for decellularization (PEG +gamma-ray 100kGy irradiation) (decellularization treatment alone), small infiltration to cells relating to rejection response (T cells or the like) was observed. In all the treatment methods from (i) -a through (ha) -b, no cell infiltration to cells relating to rejection response were not observed. Accordingly, even if the present prosthetic method was applied to tissues having properties not raising rejection response, no rejection response was raised (i.e. no rejection response was raised due to the prosthesis method).

As described above, the present invention is illustrated by way of preferred embodiments and examples, the present invention. It shouldbe understood that the scope of the present invention is limited only by the claims. It is understood that those skilled in the art can carry out equivalents of the present invention based on the description of the present invention and the common general knowledge in the art from the specific preferable embodiments of the present invention. All patents, patent applications, and publications cited in this specification are herein incorporated by reference in their entireties to the same extent as if each were specifically herein described.

INDUSTRIAL APPLICABILITY

The present invention established an improved decellularuzation technology in which tissue damage rate is suppressed to a clinically applicable level, while cell survival ratio is reduced. This technology reinforce decellularized tissue and graft prepared thereby. Therefore, such tissue is industrially applicable.

**Claims**

1. A decellularized tissue comprising a biocompatible macromolecule.

2. A Decellularized tissue according to Claim 1, wherein:

   a) a cell redisual rate of the tissue is less than a level at which an immune reaction is elicited in an organism; and
   b) the tissue is not damaged to such an extent that hinders the tissue from exhibiting a function which was possessed by the tissue when the tissue was not decellularized.

3. A decellularized tissue according to Claim 1, wherein the biocompatible macromolecule coats the tissue.

4. A decellularized tissue according to Claim 1, wherein the biocompatible macromolecule is crosslinked with the tissue.

5. A decellularized tissue according to Claim 1, wherein the biocompatible macromolecule is crosslinked with the tissue by means of a radical reaction.

6. A decellularized tissue according to Claim 1, wherein the biocompatible macromolecule is crosslinked with a irradiation selected from the group consisting of ultraviolet irradiation, exposure to a free radical source, ultrasonication, x-ray irradiation, gamma-ray irradiation and electron beam irradiation.

7. A decellularized tissue according to Claim 1, wherein the biocompatible macromolecule is biodegradable.

8. A decellularized tissue according to Claim 1, wherein the biocompatible macromolecule comprises a macromolecule selected from the group consisting of polyvinyl alcohol (PVA), polyvinyl pyrrolidone (PVP), elastin, polyethylene glycol (PEG), gelatin, collagen, gamma-polyglutaminic acid, and a mixture of the two or more thereof.

9. A decellularized tissue according to Claim 1, wherein the biocompatible macromolecule comprises polyvinyl alcohol or polyvinyl pyrrolidone.

10. A decellularized tissue according to Claim 1, wherein the polyvinyl alcohol is in the range of molecular weight of 500 to 200,000.

11. A decellularized tissue according to Claim 1, wherein the cell redisual rate of the tissue is 30% or less..

12. A decellularized tissue according to Claim 1, wherein the tissue damage rate of the tissue is 30% or less.

13. A decellularized tissue according to Claim 1, wherein the tissue has a tissue strength which permits a clinical application.

**14.** A decellularized tissue according to claim 1, wherein the tissue has a tissue strength which is 80% or more of a tissue strength which was possessed by the tissue when the tissue was not decellularized.

**15.** A decellularized tissue according to claim 1, wherein the tissue has a tissue strength having a β value which is 80% or more of a β value which was possessed by the tissue when the tissue was not decellularized.

**16.** A decellularized tissue according to claim 1, wherein the tissue has a tissue strength having a β value of 20 or more.

**17.** A decellularized tissue according to claim 1, wherein the tissue is membranous, valvular, or luminal tissue.

**18.** A decellularized tissue according to claim 1, wherein the tissue is tissue selected from blood vessels, blood vessel-like tissue, cardiac valves, pericardia, duramatter, corneas, and bones.

**19.** A decellularized tissue according to claim 1, wherein a state of the tissue, in which the tissue is not damaged to such an extent that hinders the tissue from exhibiting a function which was possessed by the tissue when the tissue was not decellularized, includes that an extracellular matrix of the tissue is not substantially degenerated.

**20.** A decellularized tissue according to claim 18, wherein a survival rate of the extracellular matrix is at least about 50%.

**21.** A decellularized tissue according to claim 1, wherein the tissue is derived from a mammal.

**22.** A Decellularized tissue according to claim 1, wherein the tissue is derived from a human or a swine.

**23.** A tissue graft comprising decellularized tissue according to Claim 1.

**24.** A tissue graft according to Claim 23, further comprising a cell.

**25.** A tissue graft according to Claim 23, wherein the tissue graft is free of a cell.

**26.** A tissue graft according to Claim 23, wherein the tissue graft has a form of selected from the group consisting of membranous, valvular, or luminal form.

**27.** A method of producing decellularized tissue, comprising the steps of:

1)providing tissue; and
2)decellularizing the tissue; and
3)exposing the tissue to a biocompatible macromolecule.

**28.** A method according to Claim 27, wherein the step of decellularizating comprises immersing the tissue in a solution containing a non-micellar amphipathic molecule or a solution containing a surfactant.

**29.** A method according to Claim 27, wherein the step of exposing the tissue to a biocompatible macromolecule comprises crosslinking the biocompatible macromolecule.

**30.** A method according to Claim 29, wherein the crosslinking comprises a radical reaction.

**31.** A method according to Claim 29, wherein the radical reaction comprises a irradiation selected from the group consisting of ultraviolet irradiation, exposure to a free radical source, ultrasonication, x-ray irradiation, gamma-ray irradiation and electron beam irradiation.

**32.** A method according to Claim 29, wherein the radical reaction is gamma-ray irradiation.

**33.** A method according to Claim 32, wherein the irradiation dose of the gamma-ray irradiation is in the range of 10-300 kGy.

**34.** A method according to Claim 32, wherein the gamma-irradiation is conducted under a circumstance selected from the group consisting of in vacuum, in oxygen, innitrogen, in the air, in water, in an amphipathic molecule solution and a combination thereof.

35. A method according to Claim 32, wherein the gamma-irradiation is conducted for between 0.5-240 hours.

36. A method according to Claim 27, wherein the biocompatible macromolecule is biodegradable.

37. A method according to Claim 27, wherein the biocompatible macromolecule comprises a macromolecule selected from the group consisting of polyvinyl alcohol (PVA), polyvinyl pyrrolidone (PVP), elastin, polyethylene glycol (PEG), gelatin, collagen, gamma-polyglutaminic acid, and a mixture of the two or more thereof.

38. A method according to Claim 27, wherein the biocompatible macromolecule comprises polyvinyl alcohol or polyvinyl pyrrolidone.

39. A method according to Claim 38, wherein the polyvinyl alcohol is in the range of molecular weight of 500 to 200,000.

40. A method according to Claim 27, wherein the biocompatible macromolecule is used at the concentration between 1 w/v% to 50 w/v%.

41. A method according to Claim 28 , wherein the amphipathic molecule is a 1,2-epoxide polymer.

42. A method according to Claim 28, wherein the amphipathic molecule is polyethylene glycol (PEG).

43. A method according to Claim 27, wherein the decellularization step is performed for 30 min to 10 days..

44. A method according to Claim 2 7, wherein the amphipathic molecule is biocompatible.

45. A method according to Claim 27, wherein the tissue is tissue selected from blood vessels, blood vessel-like tissue, cardiac valves, pericardia, dura matter, corneas, and bones.

46. A method according to Claim 27, wherein the tissue is derived from a mammal.

47. A method according to Claim 27, wherein the tissue is derived from a human or a swine.

48. A method according to Claim 27, further comprising the step of a chemical treatment which is a nuclease treatment.

49. A method according to Claim 48, wherein the chemical treatment comprises a treatment by means of DNase.

50. A decellularized tissue obtainable by a method according to Claim 27.

51. A method for regenerating a tissue, comprising the steps of:

   a)providing decellularized tissue comprising a biocompatible macromolecule into an organism; and
   b) incubating the tissue within the organism for a time sufficient for the tissue to regenerate.

52. A method according to claim 51, further comprising providing a cell to the decellularized tissue.

53. A method according to claim 51, further comprising providing a physiologically active substance which induces cellular differentiation, to the organism.

54. A method according to claim 53, wherein the physiologically active substance is from the organism or from outside the organism.

55. A method according to claim 52, wherein the physiologically active substance is provided in a form of nucleic acid or polypeptide form.

56. A method according to claim 53, wherein the physiologically active substance is selected from the group consisting of HGF, VEGF, FGF, IGF, PDGF and EGF.

57. A method according to claim 51, wherein the tissue is tissue selected from blood vessels, blood vessel-like tissue, cardiac valves, pericardia, dura mater, corneas, and bones.

**58.** A method for producing a tissue graft, comprising the steps of:

a) providing decellularized tissue comprising a biocompatible macromolecule into an organism;
b) allowing a self cell in the organism to infiltrate the decellularized tissue; and
c) incubating the tissue within the organism for a time sufficient for the cell to differentiate.

**59.** A method according to Claim 58, wherein the tissue is tissue selected from blood vessels, blood vessel-like tissue, cardiac valves, pericardia, dura matter, corneas, and bones.

**60.** A method according to Claim 58, wherein the decellularized tissue is autologous.

**61.** A method according to Claim 58, wherein the decellularized tissue is derived from a homologous host.

**62.** A method according to claim 58, wherein the decellularized tissue is derived from a heterologous host.

**63.** A method according to Claim 58, further comprising the step of:

a) providing a physiologically active substance which induces differentiation of the cell.

**64.** A method according to Claim 63, wherein the physiological active substance is a cytokine having hemopoietic activity.

**65.** A tissue graft produced by a method according to Claim 58.

**66.** A method of treating a subject requiring transplantation of tissue or an organ or treating a subject at a risk of transplantation of tissue or an organ for prophylaxis, the method comprising the steps of:

a) providing decellularized tissue comprising a biocompatible macromolecule or a tissue graft comprising the decellularized tissue into an organism; and
b) transplanting the decellularized tissue or tissue graft to a subject.

**67.** A method according to claim 66, wherein the tissue is derived from the subject.

**68.** A method according to claim 66, wherein the tissue is tissue selected from the group consisting of bloodvessels, blood vessel-like tissue, cardiac valves, pericardia, dura matter, corneas, and bones.

**69.** A method according to claim 66, wherein the subject is a mammal.

**70.** A method according to claim 66, wherein the subject is a human.

**71.** A pharmaceutical for organ transplantation, comprising:

A) decellularized tissue comprising a biocompatible macromolecule or a tissue graft comprising the decellularized tissue into an organism.

**72.** A pharmaceutical according to claim 71, wherein the tissue is tissue selected from the group consisting of blood vessels, blood vessel-like tissue, cardiac valves, pericardia, dura matter, corneas, and bones.

**73.** A pharmaceutical according to claim 71, wherein the cell is derived from a mammal.

**74.** A pharmaceutical according to claim 71, wherein the tissue is derived from a human.

**75.** A pharmaceutical according to claim 71, wherein the tissue is derivedfrom the subject requiring transplantation.

**76.** Use of decellularized tissue comprising a biocompatible macromolecule or a tissue graft comprising the decellularized tissue for manufacture of a pharmaceutical for organ transplantation.

Fig. 1

# Fig. 2

Results of tensile strength test of native canine aorta.

| Testing machine | RTC series | | | Type of testing | Extension | | |
|---|---|---|---|---|---|---|---|
| Load in full-scale | 5 kgf | | | Rated capacity of load cell | 100 N | | |
| Range of load | 40 %RO | | | Rated capacity of the extensometer | 20 cm | | |
| Range of the extensometer | unapplied | | | Test Speed | 10.0 mm/min | | |
| Recording speed off | | | | Rigidity of the testing machine | 0 mm/kgf | | |
| Midpoint (load) | · 0 | 0 | 0 | Midpoint | 0 | 50 | 60 |
| N | 0 | 0 | 0 | (extension) cm | 0 | 0 | 0 |
| Analysis of Elastic moduli | Interval | 1 | 50 | Initial length , Distance between chunks | 10 mm | | |
| | Pitch | 1 %max | | origin in extension initial load point | 0.03 N | | |
| slack correction | applied | | | Determination of rupture point | 0.5 N | | |
| Storing SS curve | ON | | | | | | |

| TestID=37 | Maximum load | Maximum load | Rupture load | Rupture load | Maximum Extension | Elastic Modulus |
|---|---|---|---|---|---|---|
| Test No. | kgf | N | kgf | N | mm | MPa |
| 1 | 0.7591 | 7.4445 | 0.5038 | 4.9404 | 27.887 | 1.0918 |
| Average | 0.7591 | 7.4445 | 0.5038 | 4.9404 | 27.887 | 1.0918 |
| JIS weighted avg | 0.7591 | 7.4445 | 0.5038 | 4.9404 | 27.887 | 1.0918 |
| Median | 0.7591 | 7.4445 | 0.5038 | 4.9404 | 27.887 | 1.0918 |
| Maximum | 0.7591 | 7.4445 | 0.5038 | 4.9404 | 27.887 | 1.0918 |

LOAD (axis: 8, 6, 4, 2, 0)
Extension (axis: 0, 10, 20, 30) mm

# Fig. 3

## Results of tensile strength test of valves by means of decellularization cell method by SDS

| Testing machine | RTC series | | | Type of testing | Extension | | |
|---|---|---|---|---|---|---|---|
| Load in full-scale | 5 kgf | | | Rated capacity of load cell | 100 N | | |
| Range of load | 40 %RO | | | Rated capacity of the extensometer | 20 cm | | |
| Range of the extensometer | unapplied | | | Test Speed | 10.0 mm/min | | |
| Recording speed off | | | | Rigidity of the testing machine | 0 mm/kgf | | |
| Midpoint (load) | 0 | 0 | 0 | Midpoint | 0 | 50 | 60 |
| N | 0 | 0 | 0 | (extension) cm | 0 | 0 | 0 |
| Analysis of Elastic moduli | Interval | 1 | 50 | Initial length  Distance between chunks | 10 mm | | |
| | Pitch | 1 %max | | origin in extension  initial load point | 0.03 N | | |
| slack correction | applied | | | Determination of rupture point | 0.5 N | | |
| Storing SS curve | ON | | | | | | |

| TestID=17 Test No. | Maximum load kgf | Maximum load N | Rupture load kgf | Rupture load N | Elastic Modulus MPa |
|---|---|---|---|---|---|
| 1 | 1.0401 | 10.200 | 1.0284 | 10.085 | 2.5168 |
| 2 | 0.7095 | 6.9574 | 0.6856 | 6.7231 | 1.4561 |
| 3 | 0.7142 | 7.0038 | 0.6339 | 6.2164 | 1.4976 |
| 4 | 0.8572 | 8.4063 | 0.8503 | 8.3387 | 1.6630 |
| 5 | 0.6693 | 6.5639 | 0.6613 | 6.4847 | 1.1928 |
| Average | 0.7981 | 7.8263 | 0.7719 | 7.5696 | 1.6653 |
| JIS weighted avg. | 0.9196 | 9.0180 | 0.9040 | 8.8649 | 2.0527 |
| Median | 0.7142 | 7.0038 | 0.6856 | 6.7231 | 1.4976 |
| Maximum | 1.0401 | 10.200 | 1.0284 | 10.085 | 2.5168 |
| SD(n-1) | 0.1529 | 1.4996 | 0.1663 | 1.6313 | 0.5050 |
| SD(n) | 0.1368 | 1.3413 | 0.1488 | 1.4591 | 0.4517 |

LOAD (vertical axis: 12.50, 10.00, 7.50, 5.00, 2.50, 0.00)

Extension (horizontal axis: 0, 5, 10, 15, 20)

# Fig. 4

**Results of tensile strength test of the decellularized tissue (1) with a coating of PVA onto an artificial valve prepared by the decellularized method using SDS**

| Testing machine | RTC series | | Type of testing | Extension | | |
|---|---|---|---|---|---|---|
| Load in full-scale | 5 kgf | | Rated capacity of load cell | 100 N | | |
| Range of load | 40 %RO | | Rated capacity of the extensometer | 20 cm | | |
| Range of the extensometer | unapplied | | Test Speed | 10.0 mm/min | | |
| Recording speed off | | | Rigidity of the testing machine | 0 mm/kgf | | |
| Midpoint (load) | 0 | 0 | 0 Midpoint | 0 | 50 | 60 |
| N | 0 | 0 | 0 (extension) cm | 0 | 0 | 0 |
| Analysis of | Interval | 1 | 50 Initial length  Distance between chunks | 10 mm | | |
| Elastic moduli | Pitch | 1 %max | origin in extension  initial load point | 0.03 N | | |
| slack correction | applied | | Determination of rupture point | 0.5 N | | |
| Storing SS curve | ON | | | | | |

| TestID=140<br>Test No. | Maximum load<br>kgf | Maximum load<br>N | Rupture load<br>kgf | Rupture load<br>N | Maximum Extension<br>mm | Elastic Modulus<br>MPa |
|---|---|---|---|---|---|---|
| 1 | 1.0359 | 10.158 | 0.8376 | 8.2140 | 21.667 | 1.2612 |
| 2 | 1.3339 | 13.082 | 1.1904 | 11.674 | 24.060 | 1.2459 |
| 3 | 1.5541 | 15.240 | 1.1569 | 11.345 | 19.507 | 1.5949 |
| 4 | 1.4570 | 14.288 | 1.2815 | 12.567 | 19.267 | 2.0703 |
| Average | 1.3452 | 13.192 | 1.1166 | 10.950 | 21.125 | 1.5431 |
| JIS weighted avg | 1.4511 | 14.231 | 1.1973 | 11.742 | 22.407 | 1.7643 |
| Median | 1.3955 | 13.685 | 1.1737 | 11.510 | 20.587 | 1.4280 |
| Maximum | 1.5541 | 15.240 | 1.2815 | 12.567 | 24.060 | 2.0703 |
| SD(n-1) | 0.2251 | 2.2071 | 0.1933 | 1.8958 | 2.2346 | 0.3866 |
| SD(n) | 0.1949 | 1.9114 | 0.1674 | 1.6418 | 1.9352 | 0.3348 |

LOAD N vs Extension mm graph

# Fig. 5

Results of tensile strength test of the decellularized tissue (2) with a coating of PVA onto an artificial valve prepared by the decellularized method using SDS

| Testing machine | RTC series | | | Type of testing | Extension | | |
|---|---|---|---|---|---|---|---|
| Load in full-scale | 5 kgf | | | Rated capacity of load cell | 100 N | | |
| Range of load | 40 %RO | | | Rated capacity of the extensometer | 20 cm | | |
| Range of the extensometer | unapplied | | | Test Speed | 10.0 mm/min | | |
| Recording speed off | | | | Rigidity of the testing machine | 0 mm/kgf | | |
| Midpoint (load) | 0 | 0 | 0 | Midpoint | 0 | 50 | 60 |
| N | 0 | 0 | 0 | (extension) cm | 0 | 0 | 0 |
| Analysis of | Interval | 1 | 50 | Initial length Distance between chunks | 10 mm | | |
| Elastic moduli | Pitch | 1 %max | | origin in extension initial load point | 0.03 N | | |
| slack correction | applied | | | Determination of rupture point | 0.5 N | | |
| Storing SS curve | ON | | | | | | |

| TestID=141 Test No. | Maximum load kgf | Maximum load N | Rupture load kgf | Rupture load N | Maximum Extension mm | Elastic Modulus MPa |
|---|---|---|---|---|---|---|
| 1 | 1.0167 | 9.9703 | 0.9436 | 9.2538 | 19.327 | 1.2964 |
| 2 | 1.6216 | 15.902 | 1.1853 | 11.623 | 19.307 | 1.8565 |
| 3 | 2.0021 | 19.634 | 1.9176 | 18.806 | 21.647 | 1.9274 |
| Average | 1.5468 | 15.169 | 1.3488 | 13.228 | 20.093 | 1.6934 |
| JIS weighted avg. | 1.8275 | 17.921 | 1.6738 | 16.414 | 20.949 | 1.8501 |
| Median | 1.6216 | 15.902 | 1.1853 | 11.623 | 19.327 | 1.8565 |
| Maximum | 2.0021 | 19.634 | 1.9176 | 18.806 | 21.647 | 1.9274 |
| SD(n-1) | 0.4970 | 4.8734 | 0.5072 | 4.9739 | 1.3453 | 0.3457 |
| SD(n) | 0.4058 | 3.9791 | 0.4141 | 4.0612 | 1.0984 | 0.2822 |

Fig. 6

PVA coating

Decellularized valve

(PVA 10%+Na$_3$BO$_3$), γ-ray 60kGy

Fig. 7

× 40

Fig. 8

Fig. 9

Decellularization  prostheses treatment

Native

SDS,tritonX-100
treatment

| Amount of γ-irradiation applied polymer | 15kGy | 60kGy |
|---|---|---|
| **PVP** | (i)-a | (i)-b |
| **PVA** | (ro)-a | (ro)-b |
| **PEG** | (ha)-a | (ha)-b |

EP 1 698 358 A1

50

Fig. 10

Fig. 11

Fig. 12

EP 1 698 358 A1

Fig. 13

## INTERNATIONAL SEARCH REPORT

| | International application No. |
|---|---|
| | PCT/JP2004/019440 |

**A. CLASSIFICATION OF SUBJECT MATTER**
  Int.Cl⁷ A61L27/40, 27/38, A61F2/06, 2/24, 2/28, C12N5/06, 5/08

According to International Patent Classification (IPC) or to both national classification and IPC

**B. FIELDS SEARCHED**

Minimum documentation searched (classification system followed by classification symbols)
  Int.Cl⁷ A61L27/40, 27/38, A61F2/06, 2/24, 2/28, C12N5/06, 5/08

Documentation searched other than minimum documentation to the extent that such documents are included in the fields searched
  Jitsuyo Shinan Koho          1922–1996   Jitsuyo Shinan Toroku Koho   1996–2005
  Kokai Jitsuyo Shinan Koho    1971–2005   Toroku Jitsuyo Shinan Koho   1994–2005

Electronic data base consulted during the international search (name of data base and, where practicable, search terms used)
  CAPLUS/BIOSIS/MEDLINE/EMBASE (STN)

**C. DOCUMENTS CONSIDERED TO BE RELEVANT**

| Category* | Citation of document, with indication, where appropriate, of the relevant passages | Relevant to claim No. |
|---|---|---|
| X | UCHIMURA, E. et al., Novel method of preparing acellular cardiovascular grafts by decellularization with poly(ethylene glycol), Journal of Biomedical Materials Research, Part A, October 2003, Vol.67A, No.3, pages 834 to 837 | 1,2,11–23, 25,26,71–76 |
| X | UEDA, Y. et al., Development of acellular tissue for cardiovascular applications, International Journal of Artificial Organs, July 2003, Vol.26, No.7, page 598 | 1,2,11–23, 25,26,71–76 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 13 April, 2005 (13.04.05) | 26 April, 2005 (26.04.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**EP 1 698 358 A1**

<table>
<tr><td colspan="2"><strong>INTERNATIONAL SEARCH REPORT</strong></td><td>International application No.<br/>PCT/JP2004/019440</td></tr>
</table>

C (Continuation).  DOCUMENTS CONSIDERED TO BE RELEVANT

| Category* | Citation of document, with indication, where appropriate, of the relevant passages | Relevant to claim No. |
|---|---|---|
| X | JP 09-122227 A  (Bio-Engineering Laboratories, Ltd.), 13 May, 1997 (13.05.97), Claims 1, 4; Par. No. [0035] & CA 2173546 A          & CN 1149498 A & EP 773032 A1          & US 5618312 A | 1,2,11-23, 25,26,71-76 |
| X | JP 2003-190192 A  (Japan Science and Technology Corp.), 08 July, 2003 (08.07.03), Claim 3; Par. No. [0019] (Family: none) | 1,2,11-23, 25,26,71-76 |
| X | Sobi OTA et al., Fibronectin-HGF Yugo Tanpaku ni yori In situ deno Jikoka o Sokushin saseru Datsusaiboka Ishu Seitaiben no Kento", Jpn.J. Thorac.Cardiovasc.Surg., Vol.51, Zokan (10 Gatsu), page 220 | 1,2,11-23, 25,26,71-76 |

Form PCT/ISA/210 (continuation of second sheet) (January 2004)

56

**INTERNATIONAL SEARCH REPORT**

| International application No. |
| --- |
| PCT/JP2004/019440 |

---

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 51-64, 66-70
   because they relate to subject matter not required to be searched by this Authority, namely:
   Claims 51 to 64 and 66 to 70 pertain to methods for treatment of the human body or animal body by surgery or therapy and thus relate to a subject matter which this International Searching Authority is not required, under the provisions of Article 17(2)(a)(i) of the PCT    (continued to extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:

See extra sheet.

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant.    Consequently, this international search report is restricted to the invention first mentioned in the claims; it is covered by claims Nos.: 1, 2, 11-23, 25, 26 and 71-76.

**Remark on Protest**        ☐ The additional search fees were accompanied by the applicant's protest.

☐ No protest accompanied the payment of additional search fees.

Form PCT/ISA/210 (continuation of first sheet (2)) (January 2004)

**INTERNATIONAL SEARCH REPORT**

International application No.

PCT/JP2004/019440

Continuation of Box No.II-1 of continuation of first sheet(2)

and Rule 39.1(iv) of the Regulations under the PCT, to search.

Continuation of Box No.III of continuation of first sheet(2)

Invention 1: claims 1, 2, 11-23, 25, 26 and 71-76,
Invention 2: claims 3-6, 27, 29-35, 40, 45-47 and 50,
Invention 3: claims 7-10 and 36-39,
Invention 4: claims 24 and 65, and
Invention 5: claims 28, 41-44, 48 and 49.

These inventions 1-5 are common to each other in being an invention relating to a decellularized tissue containing a biocompatible polymer. However, the decellularized tissue containing a biocompatible polymer is publicly known as described in the following references 1-5. Consequently, this common matter cannot be recognized as being a "special technical feature" of invention.

It appears that in the invention 2 crosslinking or coating of a tissue with a biocompatible polymer, in the invention 3 specifying of biocompatible polymer, in the invention 4 introducing of cells in a decellularized tissue, and in the invention 5 specifying of a decellularization process constitute respective "special technical features" of invention. It does not appear that among these inventions, there is a technical relationship involving one or more of the same or corresponding special technical features.

Consequently, these inventions 1-5 are not so linked with each other as to form a single general inventive concept, and it appears that the claims 1-50, 65 and 71-76 involve five inventions.

Reference 1: UCHIMURA, E. et al, Novel method of preparing acellular cardiovascular grafts by decellularization with poly(ethylene glycol), Journal of Biomedical Materials Research, Part A, October 2003, Vol.67A, No.3, p.834-837
Reference 2: UEDA, Y. et al, Development of acellular tissue for cardiovascular applications, International Journal of Artificial Organs, July 2003, Vol.26, No.7, p.598
Reference 3: JP 09-122227 A (Bio-Engineering Laboratories, Ltd.), 13 May, 1997 (13.05.97)
Reference 4: JP 2003-190192 A (Japan Science and Technology Corp.) 08 July, 2003 (08.07.03)
Reference 5: Sobi OTA et al., Fibronectin-HGF Yugo Tanpaku ni yori In situ deno Jikoka o Sokushin saseru Datsusaiboka Ishu Seitaiben no Kento", Jpn.J. Thorac.Cardiovasc.Surg., Vol.51, Zokan (10 Gatsu), p.220

Incidentally, the decellularized tissues of the above References 1, 2 contain biocompatible polymers, such as collagen being an extracellular matrix, so that it appears that the decellularized tissues per se are comprehended in the "decellularized tissue containing a biocompatible polymer".

Form PCT/ISA/210 (extra sheet) (January 2004)